# EUROPEAN PATENT SPECIFICATION

(11) **EP 3 060 119 B1**
(45) Date of publication and mention of the grant of the patent: **23.06.2021**
(21) Application number: 14856336.4
(22) Date of filing: 19.03.2014
(51) Int. Cl.: A61B 5/00, A61B 5/11

(54) **METHOD FOR SENSING A PHYSICAL ACTIVITY OF A USER**
VERFAHREN ZUR ERFASSUNG DER KÖRPERLICHEN AKTIVITÄT EINES BENUTZERS
PROCÉDÉ DE DÉTECTION D'ACTIVITÉ PHYSIQUE DE L'UTILISATEUR

(30) Priority: 21.10.2013 WO PCT/US2013/065987
(43) Date of publication of application: 31.08.2016
(73) Proprietor: Apple Inc., Cupertino CA 95014 (US)
(72) Inventor: ELY, Colin, Cupertino, CA 95014 (US); DE JONG, Erik, Cupertino, CA 95014 (US); ROTHKOPF, Fletcher R., Cupertino, CA 95014 (US); LYNCH, Stephen Brian, Cupertino, CA 95014 (US)
(74) Representative: Lang, Johannes
(86) International application number: PCT/US2014/031258
(87) International publication number: WO 2015/060894

(56) References cited:
- WO-A2-2012/135325
- US-A1- 2008 162 088
- US-A1- 2011 063 114
- US-A1- 2011 152 636
- US-A1- 2012 116 684
- US-A1- 2012 220 428
- US-A1- 2013 150 987
- US-A1- 2013 217 979
- US-A1- 2013 233 097
- US-A1- 2013 262 298
- US-B2- 7 981 057
- US-B2- 8 337 365

## Description

### Field

This relates generally to wearable sensors and, more specifically, to recognizing and tracking movements and exercises.

### Background

Sensors have been incorporated into a variety of user devices to provide enhanced functionality and new opportunities for user interaction. Motion sensors, light sensors, position sensors, magnetometers, and a variety of other sensors have been incorporated into mobile phones (e.g., smartphones), tablet computers, step counters, and other computing devices, allowing software developers to create engaging software applications ("apps") for entertainment, productivity, health, and the like. Some devices and apps have been developed to track walking, running, and other distance activities. Users can monitor such cardio training and keep track of their progress over time.

Such devices and apps, however, are limited in the types of exercise they can track. For example, step counters and distance measuring devices and apps are unable to recognize or track strength training exercises. People engaging in strength training (e.g., weight lifting and the like) may manually record workout logs in physical books or digital spreadsheets. Such tedious manual recording, however, can be unreliable, and very few people go to the effort of keeping detailed logs despite the potential benefits for progress tracking and workout optimization over time. Moreover, people engage in many exercises beyond cardio training or strength training, such as team sports, that can be significant elements of a fitness plan but are similarly tedious to record. Devices and apps are likewise unable to automatically recognize and track such physical activities, limiting their ability to provide a complete picture of user fitness.

US 2011/0152636 A1 discloses a portable device for measuring consumed calories. WO 2012/135325 A2 discloses a physical activity data collection system.

US 2008/0162088 A1 discloses a method to achieve an accurate, extremely low power state classification implementation.

US 2013/0233097 A1 discloses an apparatus and methods for the collection, processing, storage, communication and use of data generated by an array of sensors connected to a body for the purposes of monitoring and measuring physical motion.

US 2013/0217979 A1 discloses apparatuses and methods for identifying with a single, small, intelligent activity monitor a particular type of activity from among a plurality of different activities.

### Summary

The invention is defined by the independent claims. The dependent claims define advantageous embodiments.

A network of wearable sensors is disclosed that can include a first sensor configured to be worn or carried on a first part of a body and a second sensor configured to be worn or carried on a second part of the body. The network can include, or can communicate with, a mobile device that can receive sensor information from both the first and second sensors. The combined sensor information can indicate a stance of a user wearing or carrying the first and second sensors. Movement can also be sensed by the first and second sensors, and the resulting combined sensor information can be used to determine that a user is performing a particular physical activity, exercise, or the like. Recognized physical activities or exercises can be tracked and recorded throughout a workout session. Additional sensors can also be used, including sensors in a mobile device or additional sensors worn on other parts of the body. In some examples, certain sensors can be used to recognize exercise equipment to provide additional tracking data. Sensors can also include mechanisms to provide user feedback, and apps can likewise provide feedback and progress information to users in a variety of ways to enhance utility and improve the user's experience.

### Brief Description of the Drawings

FIG. 1 illustrates an exemplary system with a sensor network having multiple sensor devices that can be worn or carried on different parts of the body.
FIG. 2 illustrates an exemplary sensor device that a user can wear or carry.
FIG. 3 illustrates exemplary sensor devices configured for and placed on various parts of a body.
FIG. 4A illustrates the palm side of an exemplary glove with incorporated sensors.
FIG. 4B illustrates the back side of an exemplary glove with incorporated sensors.
FIG. 5A illustrates an exemplary wristwatch with a display that can be dimmed or disabled based on sensor information from incorporated sensors.
FIG. 5B illustrates an exemplary wristwatch with a display that can be brightened or enabled based on sensor information from incorporated sensors.
FIG. 6A illustrates an exemplary wrist sensor with haptic feedback at a first extreme of an exercise motion.
FIG. 6B illustrates an exemplary wrist sensor with haptic feedback at a second extreme of an exercise motion.
FIG. 7A illustrates an exemplary ankle sensor with haptic feedback at a first position in an exercise motion.
FIG. 7B illustrates an exemplary ankle sensor with haptic feedback at a second position in an exercise motion.
FIG. 8A illustrates free weights with exemplary weight tags that can communicate information to a sensor device.
FIG. 8B illustrates a weight machine with exemplary machine and control tags that can communication with a sensor device.
FIG. 9A illustrates an exemplary review of a tracked exercise.
FIG. 9B illustrates an exemplary fitness log based on tracked workouts.
FIG. 10 illustrates an exemplary muscle heat map indicating muscles exercised during different workouts.
FIG. 11 illustrates exemplary wrist and ankle sensors tracking body positioning of a diver during a front flip dive.
FIG. 12 illustrates an exemplary process for determining an exercise being performed by a user from sensor information.
FIG. 13 illustrates an exemplary process for determining the motions of a user through three-dimensional space from sensor information.
FIG. 14 illustrates an exemplary system for receiving and processing sensor information.
FIG. 15 illustrates an exemplary smartphone that can receive and process sensor information.
FIG. 16 illustrates an exemplary media player that can receive and process sensor information.
FIG. 17 illustrates an exemplary wristwatch that can receive and process sensor information.
FIG. 18 illustrates an exemplary tablet computer that can receive and process sensor information.

### Detailed Description

In the following description of examples, reference is made to the accompanying drawings in which it is shown by way of illustration specific examples that can be practiced. It is to be understood that other examples can be used and structural changes can be made without departing from the scope of the various examples.

This relates to a network of sensors that can be used to track the stance, position, movements, exercises, and the like of a user. One or more sensor devices can be configured for wearing, attaching, or carrying on different parts of a user's body. Sensor information gathered by the sensors can be communicated to a user device, such as a smartphone, tablet computer, central sensor device, or the like. In some examples, the user device can include sensors, and the collected sensor information from the user device can also be used. The combined sensor information can be used in a variety of ways, such as to recognize a particular exercise or physical activity from the relative movements of the sensors. Recognized physical activities or exercises can be tracked and recorded throughout a workout session and over time.

In some examples, certain sensors can be used to recognize exercise equipment to provide additional tracking data, provide aural, visual, or other sensory instructions to a user, enable user control of an exercise machine, or the like. Sensors can also include mechanisms to provide user feedback, and apps can likewise provide feedback and progress information to users in a variety of ways to enhance utility and improve the user's experience.

It should be understood that many other applications are possible using various sensors in different configurations.

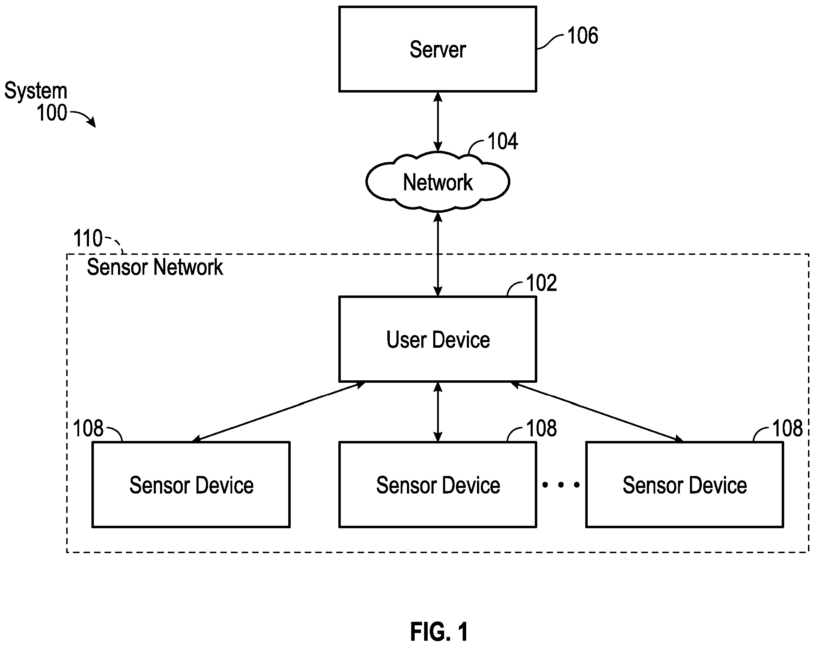

FIG. 1 illustrates exemplary system 100 with sensor network 110 having user device 102 and multiple sensor devices 108. Sensor devices 108 can include any of a variety of sensors, such as accelerometers, gyroscopes, magnetometers, humidity sensors, temperatures sensors, pressure sensors, or the like. Sensor devices 108 can also include any of a variety of transmitters, such as Bluetooth antennas, radio frequency (RF) transceivers, Wi-Fi antennas, cellular antennas, or the like for communicating to or with user device 102 or with each other. Sensor devices 108 can also include a battery to power the sensors and transmitters.

Sensor devices 108 can be configured to be carried, worn, or attached to various parts of a user's body. For example, a first sensor device 108 can be configured to be worn on a user's wrist (e.g., as a bracelet, wristwatch, wristband, gloves, etc.). A second sensor device 108 can be configured to be clipped to or inserted in a user's shoe or worn on a user's ankle (e.g., as an ankle bracelet). Still other sensor devices 108 can be configured to be carried in a shirt pocket, pant pocket, skirt pocket, or pouch; clipped to a shirt sleeve, waistband, or shoelace; worn in an armband, gloves, or headphones; or carried, worn, or attached in any of a variety of other positions around a user's body. In some examples, sensor devices 108 can be built for durability, robustness, and the like for safe operation in a variety of environments (e.g., without damaging sensors, transmitters, or other components). For example, sensor devices 108 can be configured for safe operation in any environment, including cold, hot, wet, dry, high altitude, noisy (both audible noise and potentially interfering signal noise), etc. User device 102 can also include sensors, can be built for robustness, and can similarly be configured to be carried, worn, or attached to various parts of a user's body (e.g., carried in a pocket, attached in an armband, worn as necklace, etc.).

Sensor devices 108 can gather sensor data and communicate the data to user device 102. For example, sensor devices 108 can gather sensor data related to position, movement, temperature, humidity, pressure, or the like and transmit the data to user device 102. In some examples, one sensor device 108 can transmit data to another sensor device 108, such as transmitting a heartbeat signal or ping signal to another sensor device, which can be used to determine relative position, distance, and other information (e.g., as in RF time of flight ranging). In still other examples, user device 102 and sensor devices 108 can transmit information to and receive information from any other device within sensor network 110, enabling both the transfer of sensed data as well as various measurements based on signals being sent and received (e.g., as in echo location, RF time of flight ranging, various triangulation schemes, or the like).

User device 102 can aggregate the received sensor data from sensor devices 108 and, in some examples, sense signals from sensor devices 108 that are indicative of position, distance, or the like as well as combine sensor data from sensors within user device 102. User device 102 can include a processor that can be configured to perform any of a variety of analyses on the data collected from sensor devices 108, data from sensors within user device 102, and data derived from signals generated by sensor devices 108. For example, user device 102 can determine from the combined sensor information a relative position of the various devices within sensor network 110. In some examples, from that determination, user device 102 can also determine a stance of a user wearing, carrying, or otherwise using the various devices in sensor network 110. User device 102 can include, for example, a smartphone, tablet computer, laptop computer, portable media player, or the like. In some examples, user device 102 can be a mobile device either worn or carried by the user or placed proximate to the user. In other examples, user device 102 can be a stationary device proximate to user.

User device 102 can be communicatively coupled to network 104, which can include any type of wired or wireless network, such as the Internet, a cellular network, a Wi-Fi network, a local area network (LAN), a wide area network (WAN), or the like. In some examples, user device 102 can communicate with server 106 through network 104. Server 106 can provide information or updates supporting an app on user device 102. In some examples, user device 102 can transmit collected sensor information to server 106, and server 106 can process the sensed information remotely. In other examples, sensor information can be collected and used by server 106 to improve recognition algorithms on user device 102. For example, a user can manually indicate a stance, position, exercise, movement, or the like performed while sensor devices 108 collect sensor data. The indicated stance, position, exercise, movement, or the like can then be transmitted to server 106 through network 104 along with the sensor data, and both can be aggregated and compared to prior entries of that user and/or other users. The aggregated and compared data can then be used to improve recognition algorithms (including statistical probabilities of accuracy) to allow user device 102 to automatically recognize the indicated stance, position, exercise, movement, or the like in the future. In other examples, machine learning, recognition algorithm improvement, and the like can be performed directly on user device 102 as data is collected over time.

It should be understood that system 100 can include fewer or more components than are illustrated in the example of FIG. 1. For example, in some instances, sensor network 110 can include user device 102 and a single sensor device 108 that can be used together to recognize a user's stance or movements. In other examples, three or more sensor devices 108 can be included in sensor network 110. In still other examples, the number of sensor devices 108 can be varied as desired by a user to improve accuracy, to enable additional recognition features, or the like (e.g., adding an additional sensor device 108 can improve recognition accuracy and/or allow for recognition of additional movements beyond those recognizable with fewer sensor devices). In other examples, sensor network 110 can include multiple user devices 102 that can be used by a single user or multiple users, where the user devices can communicate with each other and/or the other's sensor devices 108 either directly within the sensor network 110 or via the system network 104.

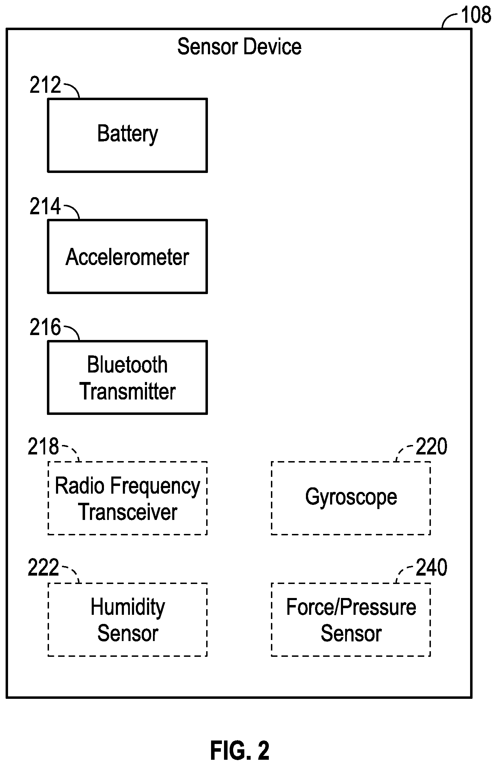

Fig. 2 illustrates exemplary sensor device 108 that a user can wear or carry in any of a variety of ways and positions as mentioned above. Sensor device 108 can belong to sensor network 110 of system 100 of FIG. 1. Sensor device 108 can include a variety of components and sensors in a variety of configurations. In some examples, different configurations of sensor device 108 can be optimized for different placement positions around a user's body (e.g., optimized for ankle placement, wrist placement, pocket placement, armband placement, etc.). In some examples, sensor device 108 can include battery 212 that can supply power to any of the other components of sensor device 108. Battery 212 can be removable and replaceable, or, in some examples, battery 212 can be rechargeable. For example, battery 212 can be recharged in any of a variety of ways, such as through wireless charging through the casing of sensor device 108, through a wall charger adapter, through a docking station, through solar panels (not shown) incorporated in sensor device 108, through linear induction charging (not shown) incorporated in sensor device 108, through mechanical crank or flywheel charging (not shown) incorporated in sensor device 108, or through any of a variety of other charging mechanisms. In other examples, any of the sensors discussed herein can include passive sensors that can generate a sensor signal in response to a signal received from another device or sensor, and such passive sensors can, in some examples, function without battery power (e.g., a passive near field communication or NFC tag).

Sensor device 108 can also include accelerometer 214. In some examples, accelerometer 214 can sense the orientation of sensor device 108 (e.g., multi-axial sensing) and generate corresponding data signals indicating the sensed orientation. Accelerometer 214 can also sense movement or acceleration of sensor device 108 and generate corresponding data signals indicative of the sensed movement or acceleration. Accelerometer 214 can include similar capabilities as accelerometers incorporated into many smartphones for orientation and motion sensing.

Sensor device 108 can also include Bluetooth transmitter 216 that can, for example, transmit information to a user device, another sensor device in a sensor network, a sensor associated with an exercise machine, a sensor associated with controllable equipment, or the like. In some examples, Bluetooth transmitter 216 (or a Bluetooth receiver) can also receive Bluetooth signals from a user device, another sensor device in a sensor network, an exercise machine, controllable equipment, or the like. In one example, orientation and motion information sensed by accelerometer 214 can be transmitted to a user device via Bluetooth transmitter 216.

Many different configurations are possible for sensor device 108, including those illustrated by dotted lines in FIG. 2. Sensor device 108 can include, for example, radio frequency transceiver 218 that can send and receive information via RF. Radio frequency transceiver 218 can be included in sensor device 108 instead of or in addition to Bluetooth transmitter 216. Radio frequency transceiver 218 can be used to perform RF time of flight ranging by sending signals and/or receiving signals that can be processed to determine distance between two devices (e.g., a distance between an ankle sensor device and a wrist sensor device). Radio frequency transceiver 218 can transmit data directly to a user device or can communicate data to Bluetooth transmitter 216 for transmission to a user device.

Sensor device 108 can also include gyroscope 220 that can be used to measure orientation, rotation, and the like. Gyroscope 220 can be included instead of or in addition to accelerometer 214. In some examples, the combination of accelerometer 214 and gyroscope 220 can allow for robust direction and motion sensing, allowing for accurate recognition of movement of sensor device 108 within a three-dimensional space (e.g., using three-dimensional coordinates, tracking displacement through three-dimensional space, etc.). Data from gyroscope 220 can be transmitted to a user device via Bluetooth transmitter 216 (or another communication mechanism, such as radio frequency transceiver 218).

Sensor device 108 can also include humidity sensor 222 (or hygrometer 222). In some examples, humidity sensor 222 can sense the humidity of the environment surrounding sensor device 108. For example, humidity sensor 222 can detect the humidity changes of an environment throughout the day, throughout a workout, or the like. In some examples, sensor device 108 can be waterproof or otherwise usable in wet conditions, and humidity sensor 222 can detect submersion in water. Similarly, humidity sensor 222 or a sensor similar to a humidity sensor can be included in sensor device 108 to detect sweat on a user's skin or even an amount of sweat accumulated on a user's skin. Humidity information from humidity sensor 222 can be transmitted to a user device via Bluetooth transmitter 216 (or another communication mechanism, such as radio frequency transceiver 218).

Sensor device 108 can also include force/pressure sensor 240. Force/pressure sensor 240 can sense an amount of force applied to a portion or all of sensor device 108. For example, sensor device 108 can be incorporated into the palm of a glove (or force/pressure sensor 240 can be incorporated into the palm of a glove with other components elsewhere on the glove), and force/pressure sensor 240 can be used to sense that a user is grasping a piece of equipment (free weights, chin-up bar, etc.). In some examples, force/pressure sensor 240 can also sense force information that can be used to determine an amount of weight being held in the palm of a user's hand. Force/pressure sensor 240 can also sense pressure applied to a portion or all of sensor device 108, or in other examples the pressure of the atmosphere surrounding sensor device 108. For example, force/pressure sensor 240 can detect pressure information that can be used to determine an altitude. Similarly, force/pressure sensor 240 can detect pressure information that can be used to determine depth of submersion in water (e.g., to determine the depth of a user diving while wearing sensor device 108). Force/pressure sensor 240 can also detect force and/or pressure that can be used to determine a user's blood pressure, heart rate or pulse, and the like. Force/pressure sensor 240 can also detect the force of an impact, such as punching an object, kicking a ball, or the like. For example, a sensor could be placed on a shoe to detect impact force upon kicking a soccer ball or the like. Force or pressure data sensed by force/pressure sensor 240 can be transmitted to a user device via Bluetooth transmitter 216 (or another communication mechanism, such as radio frequency transceiver 218).

Sensor device 108 can also include a variety of other sensors that are not illustrated in FIG. 2. For example, sensor device 108 can also include a temperature sensor that can sense the temperature of the surrounding environment and/or the temperature of a user's skin near sensor device 108. Sensor device 108 can also include a magnetometer or compass that can be used to detect the earth's magnetic field and to provide direction information. Sensor device 108 can also include a global positioning system sensor (GPS) that can triangulate the coordinate position of sensor device 108 based on sensed global positioning satellite signals. Sensor device 108 can also include a light sensor and/or a camera that can be used to detect light, take photographs, recognize a user's face, identify the direction of a user's gaze, or the like. Sensor device 108 can also include a proximity sensor that can be used to detect the presence of a user's face, objects, the ground, or the like. Sensor device 108 can also include a muscle contraction sensor that can be used to detect the contractions and orientations of a user's muscles. It should thus be understood that sensor device 108 can include various combinations of the sensors illustrated in FIG. 2 as well as a variety of other sensors that are not shown.

Sensor device 108 can also include a variety of other communication mechanisms that are not shown in FIG. 2. For example, sensor device 108 can include a cellular antenna that can send and receive information using a cellular telephone network. Sensor device 108 can also include a Wi-Fi antenna that can send and receive information using a Wi-Fi network. Sensor device 108 can also include a near field communication (NFC) radio that can communicate with other NFC radios or unpowered NFC chips called "tags." It should thus be understood that sensor device 108 can include a variety of communication mechanisms other than those illustrated in FIG. 2.

Sensor device 108 can also include a memory, such as a flash memory, hard disk drive, or the like. In some examples, sensor data can be recorded within sensor device 108 while also being transferred to a user device. In other examples, sensor data can be recorded within sensor device 108 and transferred at a later time to a user device. For example, sensor device 108 can sense and record information when outside communication range of a user device. When sensor device 108 comes within the communication range of the user device, the recorded sensor data can then be transferred to the user device automatically. In some examples, a user can wear particular sensor devices during a physical activity without carrying or wearing a user device (e.g., a smartphone). The sensor devices can record sensed data throughout the physical activity for later transmission to a user device.

It should be understood that sensor device 108 can include a variety of other components as desired in particular configurations. For example, sensor device 108 can include a display (e.g., an LCD screen), an LED indicator light, a speaker, a microphone, a camera, a light sensor, a camera flash, buttons, switches, and the like.

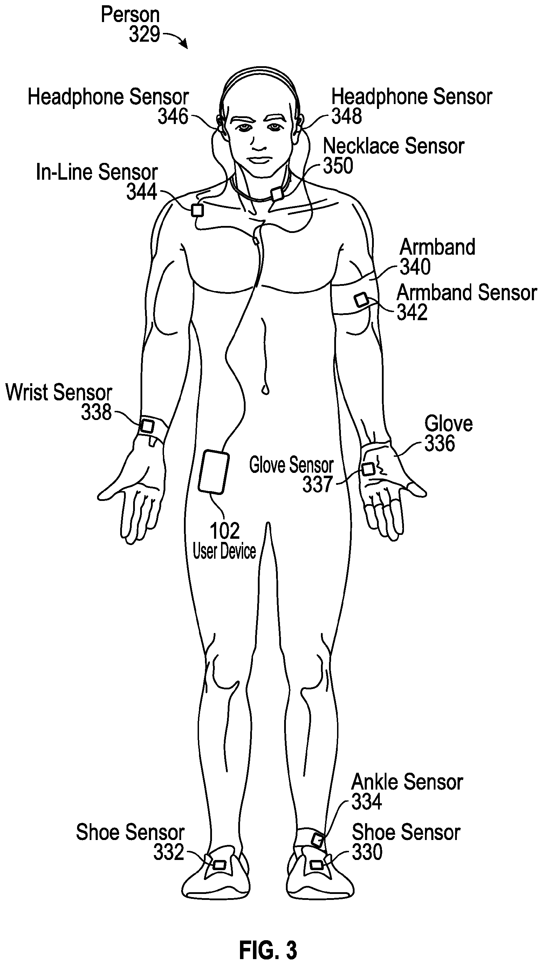

FIG. 3 illustrates exemplary sensor devices configured for and placed on various parts of a body. The various illustrated sensor devices can include any of the sensors and components illustrated and discussed with reference to sensor device 108 in FIG. 1 and FIG. 2 (e.g., any combination of various sensors and communication mechanisms). In addition, any number and any combination of the illustrated sensor devices can form part of sensor network 110 discussed above with reference to system 100 in FIG. 1. In particular, although various exemplary sensor devices and placements are illustrated, it should be understood that fewer and other devices and alternative placements are possible in configuring a sensor network that can, in one example and among other things, recognize the physical activity of the user, including stance, movements, sports activities, exercises, and the like of a user.

In one example, person 329 can carry user device 102 in a pocket, clip user device 102 to a waistband, wear user device 102 in a designated pouch, or the like. User device 102 in the illustrated example can include a smartphone, tablet computer, portable media player, or the like. In some examples, user device 102 can include any of the sensors and communication mechanisms discussed above with reference to sensor device 108. For example, user device 102 can include an accelerometer, a gyroscope, and a Bluetooth transmitter, along with other sensors and communication mechanisms. Sensor information from user device 102 can be used for a variety of purposes, such as tracking distance traversed (e.g., displacement), tracking altitude, recording the path of a user's hips through three-dimensional space over time, counting steps taken, or the like. As in system 100 discussed above, user device 102 can form part of a sensor network and can receive sensor data and other signals from various sensor devices on person 329.

In one example, a sensor network on person 329 can include shoe sensor 330 and/or shoe sensor 332. Shoe sensors 330 and 332 can be configured to clip onto shoelaces, rest inside a shoe compartment, attach to a shoe surface, attach to socks, or the like, or shoe sensors 330 and 332 can be built into shoes or particular shoe pieces. Shoe sensors 330 and 332 can include a variety of sensors, such as accelerometers and/or gyroscopes to sense movement, orientation, rotation, and the like. Sensor information from shoe sensors 330 and 332 can be used for a variety of purposes, such as determining the position and orientation of a user's foot, tracking steps, recording the path of a user's foot in three-dimensional space over time, determining distance traversed, measuring velocity, or the like.

A sensor network on person 329 can also include ankle sensor 334. Although a single ankle sensor 334 is shown in FIG. 3, it should be understood that two ankle sensors (e.g., one on each ankle) can be used in some examples. Ankle sensor 334 can be configured as part of an ankle bracelet, ankle band, chain, or the like, or ankle sensor 334 can be configured to be clipped onto or attached to ankle bracelets, ankle bands, chains, socks, shoes, pant legs, or the like. Ankle sensor 334 can include a variety of sensors, such as accelerometers and/or gyroscopes to sense movement, orientation, rotation, and the like. Sensor information from ankle sensor 334 can be used for a variety of purposes, such as determining the position and orientation of a user's leg, tracking steps, recording the path of a user's leg in three-dimensional space over time, determining distance traversed, measuring velocity, or the like.

A sensor network on person 329 can also include glove sensor 337, which can be incorporated into glove 336. Although a single glove 336 is shown in FIG. 3, it should be understood that two gloves (e.g., one on each hand) can be used in some examples. Similarly, although a single glove sensor 337 is shown on glove 336, it should be understood that multiple sensors can be incorporated into or attached to glove 336 (e.g., sensors on the palm side, back side, around the wrist, near the fingers, etc.). Glove 336 can include, for example, a weight lifting glove or the like. Glove sensor 337 can include a variety of sensors, such as accelerometers, gyroscopes, force/pressure sensors, humidity sensors, and the like. Sensor information from glove sensor 337 can be used for a variety of purposes, such as approximating an amount of weight held in a user's hand, sensing that a user is grasping a piece of equipment, sensing the orientation of a user's hand relative to the user's body, recording the path of a user's hand in three-dimensional space over time, measuring velocity of hand movement, reading data from a nearby sensor tag, sending commands to controllable equipment, measuring a user's blood pressure, measuring a user's heart rate or pulse, or the like. Moreover, glove sensor 337 can include additional components and features as desired, such as a screen, buttons, lights, microphone, speaker, camera, or the like.

A sensor network on person 329 can also include wrist sensor 338. Wrist sensor 338 can include similar sensors for similar purposes as glove sensor 337. In some examples, wrist sensor 338 can include the same or similar sensors as glove sensor 337, but attached to a wristband or the like instead of a glove. Wrist sensor 338 can be incorporated into a wristwatch, wristband, bracelet, chain, shirt sleeve, or the like, or wrist sensor 338 can be configured to be attached to a wristwatch, wristband, bracelet, chain, shirt sleeve, or the like near the wrist or hand. Although a single wrist sensor 338 is shown in FIG. 3, it should be understood that two wrist sensors can be used in some examples (e.g., one on each wrist), or a wrist sensor 338 on one hand can be used in conjunction with a glove sensor 337 on the other hand as depicted.

Wrist sensor 338 can include a variety of sensors, such as accelerometers, gyroscopes, force/pressure sensors, humidity sensors, and the like. Sensor information from wrist sensor 338 can be used for a variety of purposes, such as sensing the orientation of a user's hand relative to the user's body, recording the path of a user's wrist in three-dimensional space over time, measuring velocity of wrist movement, reading data from a nearby sensor tag, sending commands to controllable equipment, measuring a user's blood pressure, measuring a user's heart rate or pulse, or the like. Moreover, wrist sensor 338 can include additional components and features as desired, such as a screen, buttons, lights, microphone, speaker, camera, or the like. For example, wrist sensor 338 can provide additional functionality for a user beyond sensing, such as displaying a clock, displaying information, giving audible feedback, giving haptic feedback, or the like.

A sensor network on person 329 can also include armband sensor 342. Although a single armband sensor 342 is shown in FIG. 3, it should be understood that two armband sensors (e.g., one on each arm) can be used in some examples. In one example, armband sensor 342 can be configured as part of armband 340. In other examples, armband sensor 342 can be configured to be attached to or incorporated into a shirt sleeve, portable device armband pouch, or the like. Armband sensor 342 can include a variety of sensors, such as accelerometers, gyroscopes, humidity sensors, force/pressure sensors, or the like. Sensor information from armband sensor 342 can be used for a variety of purposes, such as determining the position and orientation of a user's arm, tracking arm swings, recording the path of a user's arm through three-dimensional space over time, determining distance traversed, measuring velocity, measuring muscle contractions, measuring a user's blood pressure, measuring a user's heart rate or pulse, monitoring sweat production, monitoring temperature, or the like.

A sensor network on person 329 can also include necklace sensor 350. Necklace sensor 350 can be incorporated into or attached to a necklace, neckband, chain, string, or the like. Necklace sensor 350 can include a variety of sensors, such as accelerometers, gyroscopes, temperature sensors, force/pressure sensors, microphones, or the like. Sensor information from necklace sensor 350 can be used for a variety of purposes, such as determining a user's heart rate or pulse, monitoring sweat production, monitoring temperature, recording the path of a user's neck through three-dimensional space over time, determining distance traversed, measuring velocity, or the like.

A sensor network on person 329 can also include sensors incorporated into a set of headphones that can be attached to or in communication with user device 102. For example, a set of headphones can include in-line sensor 344, headphone sensor 346, and headphone sensor 348. In-line sensor 344 can be configured as part of a set of headphones in line with headphone cables (e.g., similar to in-line microphones and volume controls), or in-line sensor 344 can be configured to be attached to or clipped onto a headphone cable. Headphone sensors 346 and 348 can be incorporated into the earpieces of a set of headphones, or headphone sensors 346 and 348 can be configured to attach to or clip onto earpieces or headphone cables near earpieces.

In-line sensor 344 and headphones sensors 346 and 348 can include a variety of sensors, such as accelerometers, gyroscopes, temperature sensors, force/pressure sensors, microphones, or the like. Sensor information from in-line sensor 344 and headphones sensors 346 and 348 can be used for a variety of purposes, such as determining a user's heart rate or pulse, monitoring sweat production, monitoring temperature, recording the path of a user's head through three-dimensional space over time, determining distance traversed, measuring velocity, determining the orientation of a user's head, determining the line of sight or visual field of a user's eyes, or the like.

It should be understood that other sensors and placements are possible that can form part of a sensor network. For example, sensors can be positioned on the back to monitor posture or back positions during a lift, gymnastic routine, or the like. Moreover, any of the sensors in FIG. 3 can be duplicated or repositioned depending on desired applications. In one example, sensors can be configured for a particular placement as depicted in FIG. 3 (e.g., ankle, wrist, arm, etc.). In other examples, one sensor can be configured to be positioned in a variety of positions around a user's body. For example, ankle sensor 334 can also be configured for use as glove sensor 337, armband sensor 342, or necklace sensor 350. In one example, a user can place multiple sensors as desired, and user device 102 can automatically determine the placement of the sensors based on sensing typical user movements or other sensor data during, for example, a calibration period (e.g., recognizing a shoe sensor from sensing typical walking motions, recognizing a wrist sensor from sensing typical arm swinging motions, recognizing a necklace sensor from sensing typical neck motions while walking, etc.).

In other examples, a user can indicate through an app on user device 102 or through buttons or switches on the various sensors where different sensors are placed (e.g., by manually indicating the placement of sensors that are sensed as forming part of a sensor network). For example, a sensor can have switches, buttons, lights, or the like for enabling a user to manually indicate where a sensor is to be placed on a body (e.g., shoe, ankle, wrist, palm, finger, neck, arm, hip pocket, back pocket, waistband, ear, shoulder, etc.). In other examples, an app on user device 102 can display a list of sensors that are sensed nearby (e.g., indicating sensor identification numbers or the like), and a user can indicate via the app the placement of each of the listed or desired sensors. It should be understood that other methods are possible for recognizing or indicating sensor placement.

Moreover, in some examples, the number and placement of sensors can be varied based on desired functionality. For example, a user can opt to use one ankle sensor (or two ankle sensors) to monitor the user's gait and record the path of the user's ankle through three-dimensional space during a walk or run. In a different example, a user can opt to use one or two ankle sensors in combination with one or two wrist sensors to record the path of the user's feet and hands throughout karate or dance routines.

In some examples, user device 102 can be configured to collect sensor data to automatically recognize and track user activity, such as automatically recognizing and tracking a user's strength training exercises during a workout. Different sensors in different places can be desirable for enabling user device 102 to automatically recognize and track a user's physical activities and exercises. For example, to recognize that a user is performing a push-up, a sensor can be desirable near the head, neck, or core in addition to a sensor on a wrist or ankle to sense an increasing distance from the ground during the up motion and a decreasing distance during the down motion, as well as to sense that a user is in a prone position during the activity. Similarly, to recognize that a user is performing jumping jacks, sensors can be desirable on a wrist and on an ankle to recognize the inward and outward motions of the legs as well as the up and down arc of the arms, as well as to sense that a user is in a standing position during the activity. It should thus be understood that the number and placement of sensors can be varied as desired based, for example, on desired functionality.

Any of a variety of exercises and physical activities can be automatically recognized and/or tracked using a sensor network as discussed herein. For example, a sensor network that includes sensors near a user's wrist, ankle, head, and waist can be used to automatically recognize and track a wide variety of strength training exercises, such as chin ups, pull ups, dips, lateral pull-downs, overhead shoulder presses, bent-over barbell rows, bent-over dumbbell rows, upright rows, cable rows, barbell bench presses, dumbbell bench presses, pushups, squats, lunges, deadlifts, power cleans, back extensions, and the like. In one example, typical sensor data corresponding to each physical activity or exercise can be stored in a database. Collected sensor information can then be compared to stored database activities to determine which physical activity or exercise a user is performing.

In some examples, machine learning techniques can be used to improve the accuracy of activity recognition from sensor data. User data can be aggregated and compared, and recognition algorithms can be improved over time as additional data is gathered and processed. Similarly, users can manually indicate the physical activity being performed to train a user device to automatically recognize the activity in the future (e.g., entering the name of a particular martial arts movement that may not yet be in the database). Multiple users can also contribute to the development and improvement of a database over time by correlating collected sensor data with particular physical activities to train the database. It should be understood that still other methods are possible for training a user device to automatically recognize and track various activities.

In addition, an app on a user device can provide a variety of functions using sensor information from a sensor network. For example, an app can use sensor information from a sensor network to automatically maintain a workout exercise log that can include such details as timing, repetitions, sets, weights, and the like associated with particular activities. A user's speed and distance can also be tracked and recorded for lifts, kicks, punches, and the like. A record of muscles exercised recently can also be kept to, for example, aid users in planning and optimizing workouts. A user's form, posture, or the like in performing certain exercises or movements can also be monitored, such as monitoring how well a user is performing a particular stretch, lift, dance move, karate move, yoga move, yoga pose, punch, kick, or the like.

The amount of power and work a user has exerted can also be tracked based on received sensor information. In some examples, a three-dimensional recording of a user's movements can be derived from sensor information, such as recording a sporting activity, dance routine, kick, lift, throw, or the like in three dimensions (e.g., using three-dimensional coordinates, displacement through three-dimensional space, etc.). Haptic feedback can also be incorporated as part of an app to direct a user's movements, indicate timing, indicate repetitions, indicate optimal form, teach a user moves, or the like through vibrations or other feedback from a user device or sensor. The amount of weight lifted and/or the equipment used throughout a workout can also be tracked, and in some examples, an app can automatically configure controllable equipment as desired for a particular workout or activity. It should thus be appreciated that many other features and functions are possible using sensor information from a sensor network.

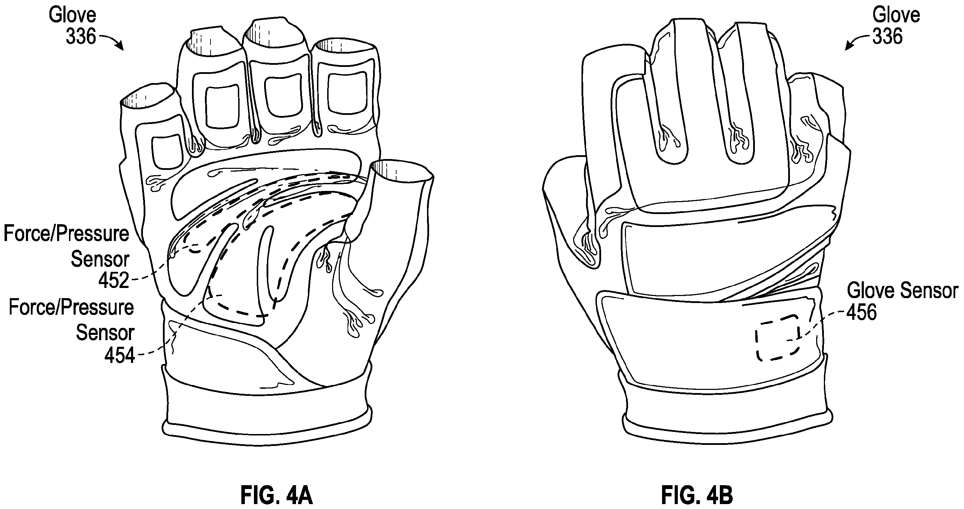

FIG. 4A illustrates the palm side of exemplary glove 336 with incorporated sensors 452 and 454, and FIG. 4B illustrates the back side of exemplary glove 336 with incorporated sensor 456. Glove 336 can include a weight lifting glove, boxing glove, or the like. Glove 336 can include force/pressure sensors 452 and 454 on the palm side as well as glove sensor 456 on the back side, or it can include fewer or additional sensors and components as desired for particular applications and functions. In one example, force/pressure sensors 452 and 454 can sense force or pressure applied to the primary impact points of weights or equipment on glove 336. The sensed force or pressure can be used to determine and record an amount of weight that is being lifted. For example, as a user lifts a weight during a bicep curl or a similar activity, force/pressure sensors 452 and 454 can sense the amount of force or pressure applied to the impact areas of glove 336 to determine the amount of weight that the user is lifting. An app on a user device in communication with force/pressure sensors 452 and 454 can track and record a user's weight lifting activity based on this sensed information (e.g., including the amount of weight, number of repetitions, speed, rest periods, and the like).

In addition to or instead of force/pressure sensors 452 and 454, glove 336 can include (incorporated into the glove or otherwise attached to the glove) glove sensor 456. Glove sensor 456 can include similar sensors and perform similar functions as glove sensor 337 discussed above with reference to FIG. 3. For example, glove sensor 456 can include accelerometers, gyroscopes, force/pressure sensors, humidity sensors, and the like. Sensor information from glove sensor 456 can be used for sensing the orientation of a user's hand relative to the user's body, recording the path of a user's hand in three-dimensional space over time, measuring velocity of hand movement, reading data from a nearby sensor tag, sending commands to controllable equipment, measuring a user's blood pressure, measuring a user's heart rate or pulse, or the like. Glove sensor 456 can also include additional components (not shown) to provide additional functions and features for a user, such as a screen, buttons, lights, microphone, speaker, camera, or the like that can be integrated into glove 336 or glove sensor 456 (which can be attached to glove 336).

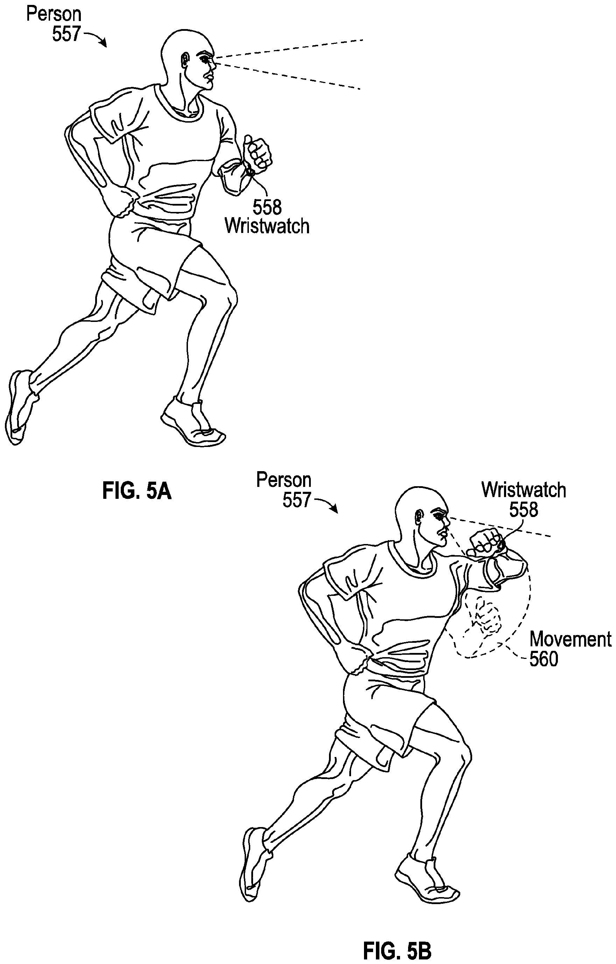

FIG. 5A illustrates exemplary wristwatch 558 with a display that can be dimmed or disabled based on sensor information from incorporated sensors, and FIG. 5B illustrates exemplary wristwatch 558 with a display that can be brightened or enabled based on sensor information from the incorporated sensors. Wristwatch 558 can include any of the sensor devices and sensors discussed herein, including a wrist sensor configured as a wristwatch (e.g., as in wrist sensor 338 of FIG. 3). In one example, sensor information from sensors in wristwatch 558 can be used to brighten or enable a display, or conversely to dim or disable a display. For example, wristwatch 558 can include an accelerometer, gyroscope, and the like for sensing motion, orientation, rotation, and the like. Sensors in wristwatch 558 can sense, for example, that person 557 is standing with arms down to the side or running with arms swinging as illustrated in FIG. 5A. In particular, sensors in wristwatch 558 can sense that the wrist of person 557 is swinging, held down to the side, angled away from the body, or the like. In such a position, wristwatch 558 can disable or dim an associated display or touchscreen under the assumption that person 557 is not looking at wristwatch 558. In other examples, wristwatch 558 can disable buttons, switches, or other interface elements to prevent accidental presses when a user is not actively interacting with the wristwatch, as inferred from the sensed position, orientation, or the like.

On the other hand, as illustrated in FIG. 5B, when person 557 raises his arm and orients wristwatch 558 toward his face, the sensors in wristwatch 558 can sense movement 560 (swinging the arm high and close to the face) as well as sense the orientation of wristwatch 558 toward the body. In such a position with such sensed information, wristwatch 558 can automatically enable or brighten an associated display or touchscreen. In other examples, wristwatch 558 can enable buttons, switches, or other interface elements to allow for previously disabled user interaction.

In some examples, a camera can be included in wristwatch 558 instead of or in addition to other sensors, and the camera can sense, for example, that a user is looking away from the wristwatch. For example, as illustrated in FIG. 5A by dotted lines, person 557 may be looking forward with a line of sight or visual field primarily forward of the body while wristwatch 558 is held down to the sides. In such a position, a camera incorporated into wristwatch 558 can sense the absence of a face or eyes near the camera. On the other hand, when person 557 raises his arm, angles wristwatch 558 toward his face, and angles his face and/or eyes toward wristwatch 558 (as illustrated by dotted lines in FIG. 5B), the camera incorporated into wristwatch 558 can sense the presence of a face or eyes near the camera in the camera's field of view. When a face and/or eyes are not detected in the camera's field of view (as in FIG. 5A), any display, touchscreen, buttons, switches, or the like can be disabled. When a face and/or eyes are detected in the camera's field of view (as in FIG. 5B), wristwatch 558 can automatically enable or brighten an associated display or touchscreen, or in other examples can enable buttons, switches, or other interface elements to allow for previously disabled user interaction. In some examples, a proximity sensor can also be used in conjunction with or instead of a camera to perform proximity sensing to aid in determining whether to enable or disable interface elements.

In other examples, the line of sight or field of view of person 557 can be determined using sensors attached to the head, and that information can be used to enable or disable a display, touchscreen, or other interface elements on wristwatch 558. For example, person 557 can wear headphones with incorporated sensors (such as headphone sensors 346 and 348 of FIG. 3). The headphone sensors can sense the orientation of the head. When the sensors detect that the head is directed forward, the sensed information can be used alone or in conjunction with other sensor information to determine that a display or other interface element can be disabled. When the sensors detect that the head is angled downward, the sensed information can be used alone or in conjunction with other sensor information to determine that a display or other interface element can be enabled. It should thus be understood that a variety of sensors can be used to determine when to enable or disable a display, touchscreen, or other interface elements on an exemplary wristwatch with incorporated sensors. It should further be understood that such enabling and disabling functions can be used for other sensor devices and sensors on other parts of the body (e.g., an armband).

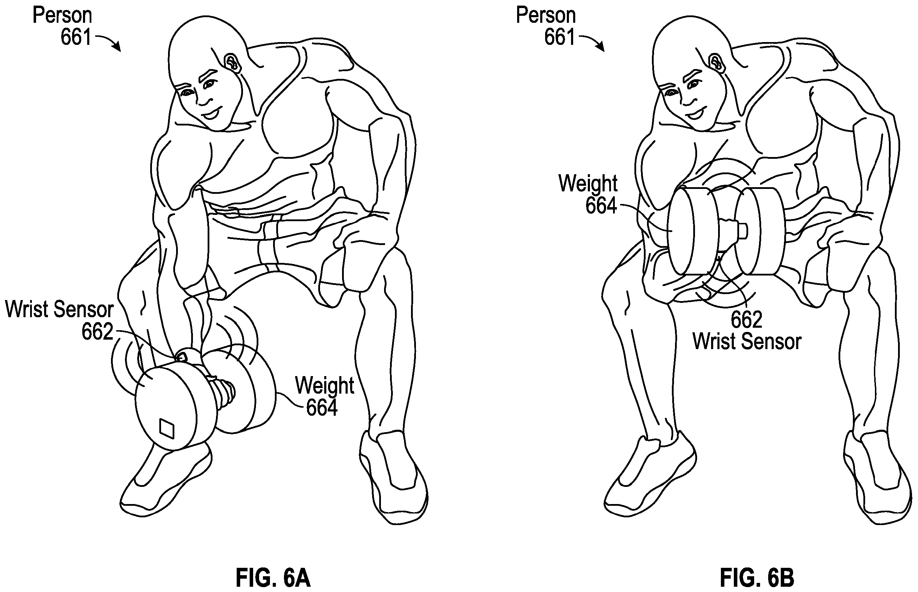

FIG. 6A and FIG. 6B illustrate exemplary wrist sensor 662 providing haptic feedback to person 661 at a first extreme of an exercise motion and at a second extreme of an exercise motion, respectively. Any of the various sensors discussed herein can include vibrators, shakers, buzzers, other mechanical stimulators, lights, speakers, or the like for providing tactile, visual, and/or aural feedback to a user. User feedback can be provided in a variety of situations to improve a user experience, aid a user in performing an exercise, direct a user to take certain actions, indicate a count, indicate a time, warn a user that further movement could lead to injury, or the like. For example, user feedback can be used to direct a user's motions during exercise, such as indicating optimal extreme positions of a motion, or to indicate status of a set of repetitions, such as indicating the end of a set, or the like.

In one example, wrist sensor 662 (or any other sensor discussed herein) can include a vibrator to provide haptic feedback. Person 661 can be engaged in any type of exercise or motion, such as a seated focused bicep dumbbell curl where person 661 lifts weight 664 from a lower extreme position as in FIG. 6A to an upper extreme position as in FIG. 6B. In one example, wrist sensor 662 can recognize alone or in conjunction with other sensors that person 661 is engaged in a bicep curl (automatically, as part of an exercise routine, as manually indicated by person 661, or the like). Wrist sensor 662 can then provide feedback during the exercise in a variety of ways. For example, at the lower extreme illustrated in FIG. 6A, wrist sensor 662 can vibrate briefly or in a particular vibration pattern to indicate that person 661 has extended his arm to an optimal position at that lower extreme of the motion. At the upper extreme illustrated in FIG. 6B, wrist sensor 662 can also vibrate briefly or in another particular vibration pattern to indicate that person 661 has curled his arm to an optimal position at that upper extreme of the motion.

In other examples, wrist sensor 662 can provide feedback during the illustrated exercise for a variety of other purposes. For example, wrist sensor 662 can vibrate briefly or in a particular vibration pattern to aid person 661 in keeping a particular rhythm, pace, or timing of curl motions. In another example, wrist sensor 662 can vibrate briefly or in a particular vibration pattern to indicate that person 661 has completed a predetermined set of repetitions or to indicate progress during a set of repetitions (e.g., a brief vibration indicating completion of half of a set and a longer vibration indicating completion of the set). In yet another example, wrist sensor 662 can vibrate briefly or in a particular vibration pattern to indicate that person 661 is within or outside of a target heart rate zone. It should thus be understood that wrist sensor 662, and any other sensor discussed herein, can provide user feedback for a variety of purposes to aid users during exercises or other physical activities. It should likewise be understood that feedback can be provided in a variety of ways other than vibration, such as blinking lights or emitting sounds.

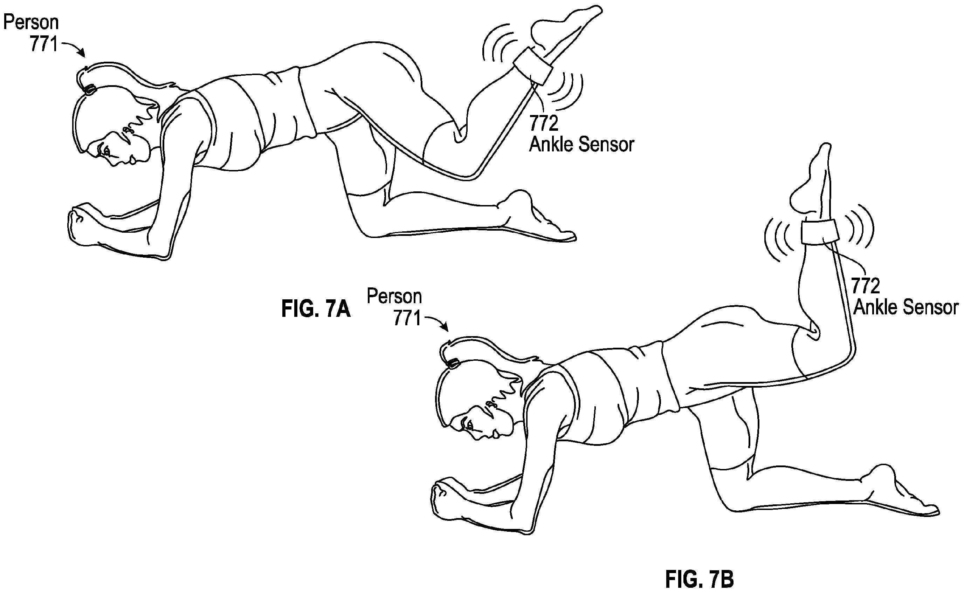

FIG. 7A and FIG. 7B illustrate exemplary ankle sensor 772 providing haptic feedback to person 771 at a first position of an exercise motion and at a second position of an exercise motion, respectively. As with wrist sensor 662 of FIG. 6A and FIG. 6B, ankle sensor 772 can include vibrators, shakers, buzzers, other mechanical stimulators, lights, speakers, or the like for providing tactile, visual, and/or aural feedback to a user. In the example illustrated in FIG. 7A and FIG. 7B, ankle sensor 772 can include a vibrator to provide haptic feedback. Person 771 can be engaged in a glute kickback exercise where person 771 assumes a kneeling pushup position and raises and lowers her leg repeatedly. In one example, ankle sensor 772 can recognize alone or in conjunction with other sensors that person 771 is engaged in a glute kickback exercise. Ankle sensor 772 can then provide feedback during the exercise in a variety of ways. For example, in a middle position of the exercise illustrated in FIG. 7A, ankle sensor 772 can vibrate briefly or in a particular vibration pattern to indicate that person 771 should slow her pace. At the upper extreme of the exercise illustrated in FIG. 7B, ankle sensor 772 can vibrate to indicate that person 771 has raised her leg to an optimal extreme of the motion.

In other examples, ankle sensor 772 can provide feedback during the illustrated exercise for a variety of other purposes. For example, ankle sensor 772 can vibrate briefly or in a particular vibration pattern to aid person 771 in keeping a particular rhythm or timing of kickback motions. In another example, ankle sensor 772 can vibrate briefly or in a particular vibration pattern to indicate that person 771 has completed a predetermined set of repetitions or to indicate progress during a set of repetitions (e.g., a brief vibration indicating completion of half of a set and a longer vibration indicating completion of the set). In yet another example, ankle sensor 772 can vibrate briefly or in a particular vibration pattern to indicate that person 771 is within or outside of a target heart rate zone. It should thus be understood that ankle sensor 772, and any other sensor discussed herein, can provide user feedback for a variety of purposes to aid users during exercises or other physical activities. It should likewise be understood that feedback can be provided in a variety of ways other than vibration, such as blinking lights or emitting sounds.

It should further be understood that the examples of FIGS. 6A, 6B, 7A, and 7B are illustrative, and any type of exercise could benefit from user feedback. It should likewise be understood that multiple sensors can function cooperatively to provide feedback to a user. For example, feedback can be provided via an armband sensor based on motions primarily sensed by an ankle sensor. Similarly, feedback can be provided aurally via headphones based on motions sensed by a shoe sensor. In addition, feedback can be used to direct users in still other ways beyond performing exercise motions, such as training a user to perform a dance routine by directing a user's motions during the routine or the like.

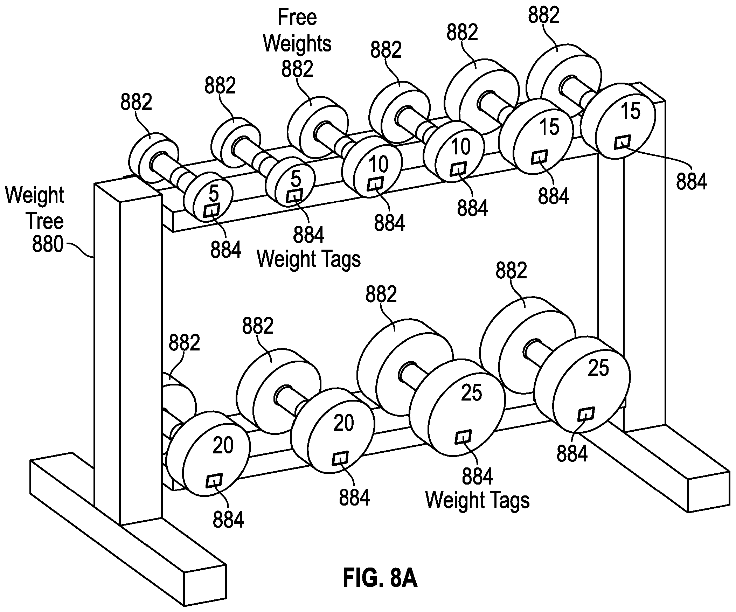

FIG. 8A illustrates free weights 882 on weight tree 880 with exemplary weight tags 884 that can communicate information to a sensor device. In some examples, any of the sensors and/or user devices discussed herein can communicate with sensors or tags that can be mounted or positioned in particular locations, on particular equipment, or the like, such as weight tags 884 mounted to free weights 882. Such sensors or tags can include any of a variety of communication mechanisms that can be active or passive. For example, tags can include active or passive NFC tags that can be stimulated by an NFC reader to produce a signal that can then be read by the NFC reader. In another example, tags can include Wi-Fi, RF, or Bluetooth tags or devices that can receive a request for information and transmit the corresponding information in response. In yet another example, tags can include barcodes, quick response (QR) codes, images, symbols, numbers, or the like that can be read using a camera and corresponding software to recognize the encoded information (e.g., a QR code scanning app or the like). For example, an app can recognize a textual number on the end of a free weight from a camera image without a separate tag. It should be understood that many other tags and devices can be used that can communicate requested information in any of a variety of ways.

In the example illustrated in FIG. 8A, any of the sensors or devices discussed herein can communicate with weight tags 884 mounted on free weights 882. Weight tags 884 can be constructed, printed, or programmed to indicate the corresponding weight of the free weight 882 to which it is attached. For example, a weight tag attached to a five pound weight (indicated by "5") can indicate that the free weight is five pounds while a weight tag attached to a twenty pound weight (indicated by "20") can indicate that the corresponding free weight is twenty pounds. In some examples, weight tags 884 can be permanently constructed or programmed to indicate a particular weight, such that they can be applied to the corresponding weights by a user or gym personnel. In other examples, weight tags 884 can be reprogrammable such that a user or gym personnel can program weight tags 884 to correspond to a particular weight as desired.

In one example, a user wearing any of a variety of sensors can engage in exercises using free weights 882. As a user removes a particular weight for use, one or more sensors associated with the user can read the weight tag 884 to recognize the amount of weight being used. The recognized amount of weight can be automatically tracked and recorded as part of an exercise log as the user's sensors and user device track and record exercises completed. In some examples, a user can scan a weight tag 884 prior to use by pointing a camera at the tag, positioning a sensor near the tag, or the like. In other examples, sensors discussed herein can scan for nearby tags automatically and track the use of the nearest tag or tags. For example, a wrist sensor can automatically detect and recognize weight tag 884 as the corresponding weight is held in a user's hand. In still other examples, weight tags 884 can be mounted on weight tree 880 and read as a user removes a weight from the corresponding position on the tree.

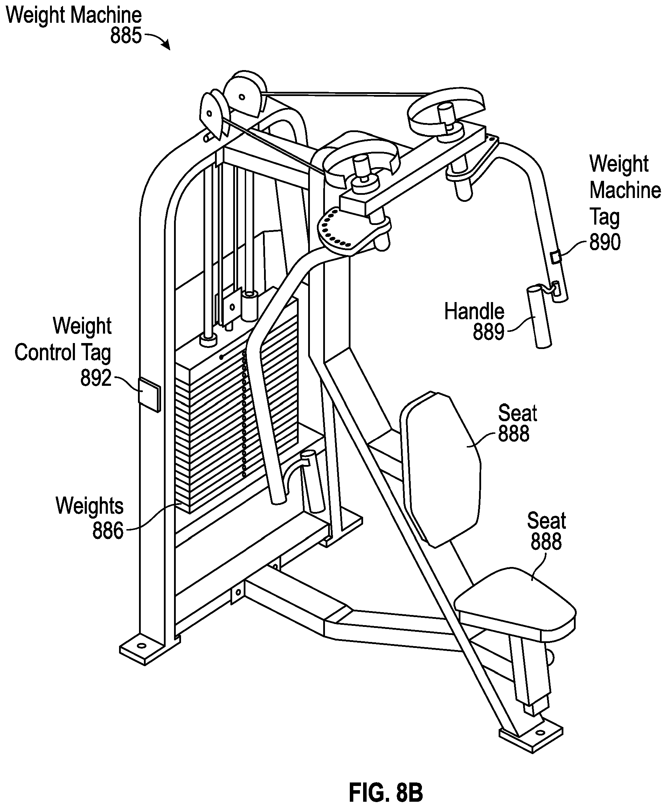

FIG. 8B illustrates another exemplary use of tags similar to weight tags 884 of FIG. 8A. FIG. 8B illustrates weight machine 885 including seat 888, handle 889, and adjustable weights 886 for performing a chest fly exercise. Weight machine 885 can have associated therewith weight machine tag 890 and/or weight control tag 892. Tags 890 and 892 can include similar communication features discussed above, such that they can communicate with any of the sensors or user devices discussed herein.

In one example, weight machine tag 890 can function in a similar fashion as weight tags 884 of FIG. 8A. In particular, weight machine tag 890 can communicate information to a user device or sensors concerning weight machine 885. For example, weight machine tag 890 can indicate that weight machine 885 is a chest fly exercise machine. A user device and sensors can then track a user's exercises near tag 890 and automatically recognize the user's movements as chest fly exercises. Similarly, a recognition algorithm on a user device used for recognizing particular exercises from user movements can take into account the information from weight machine tag 890 in determining which exercise a user is performing for tracking purposes.

In another example, weight machine tag 890 can communicate that weight machine 885 is a chest fly exercise machine, and the user's device or sensors can provide feedback or information to the user related to machine 885. For example, when a user device or sensor detects weight machine tag 890 and receives information identifying weight machine 885 as a chest fly exercise machine, the user device can cause machine instructions, tips, or the like to be played via a user's headphones. In another example, a record of past interaction with the machine can be provided to the user, such as audibly announcing to the user or displaying the amount of weight, repetitions, sets, or the like from the user's previous use or uses of machine 885. Still other information and feedback can be automatically provided to the user upon recognizing weight machine 885 based on weight machine tag 890. It should be understood that the placement of weight machine tag 890 can be varied as desired, and placing it near handle 889 is just one example that could, for example, be convenient for sensing by a wrist sensor or armband sensor.

Weight machine 885 can also have weight control tag 892 instead of or in addition to weight machine tag 890. In one example, weight control tag 892 can perform similar functions as weight machine tag 890, but can also receive requests from a user device or sensor and control weight machine 885 based on the received requests. Weight control tag 892 can include an active communication mechanism that can both receive data and send data (e.g., receive a request and send back a confirmation). For example, weight control tag 892 can establish communication with a sensor or user device and enable a user to control certain controllable features of weight machine 885 via the user device or sensors. In one example, weight control tag 892 can change the amount of weight selected on machine 885, can raise or lower seat 888, can adjust handle 889 and its associated arms back and forth, or the like. Such adjustments can be memorized from a user's previous uses of machine 885, can be entered via an interface on a user device or sensor, can be part of a workout program, or the like. In this manner, weight machine 885 can be automatically adjusted and prepared for a particular user once communication is established between weight control tag 892 and a user device or sensors. As with weight machine tag 890, the user's subsequent exercises can then be tracked and recorded as part of an exercise log.

Although a particular weight machine is illustrated in FIG. 8B, it should be understood that any weight machine or other controllable or sensory equipment can have associated therewith a control tag that can interact with a user device and/or sensors to enable a user to receive information from and/or control the equipment through the user device and/or sensors. For example, in another exemplary application, a gymnastic mat can include a communication tag and sensors for detecting a gymnast's steps during a routine and transmitting the information to a user device.

It should thus be understood that active or passive tags or devices can be placed in a variety of locations for a variety of purposes, including receiving information about a particular piece of equipment, receiving sensed information from the equipment, or controlling a piece of equipment. It should also be understood, however, that such tags can be used for any of a variety of equipment beyond exercise machines and exercise applications, such as kitchen machines, entertainment equipment, vehicle interfaces, or the like.

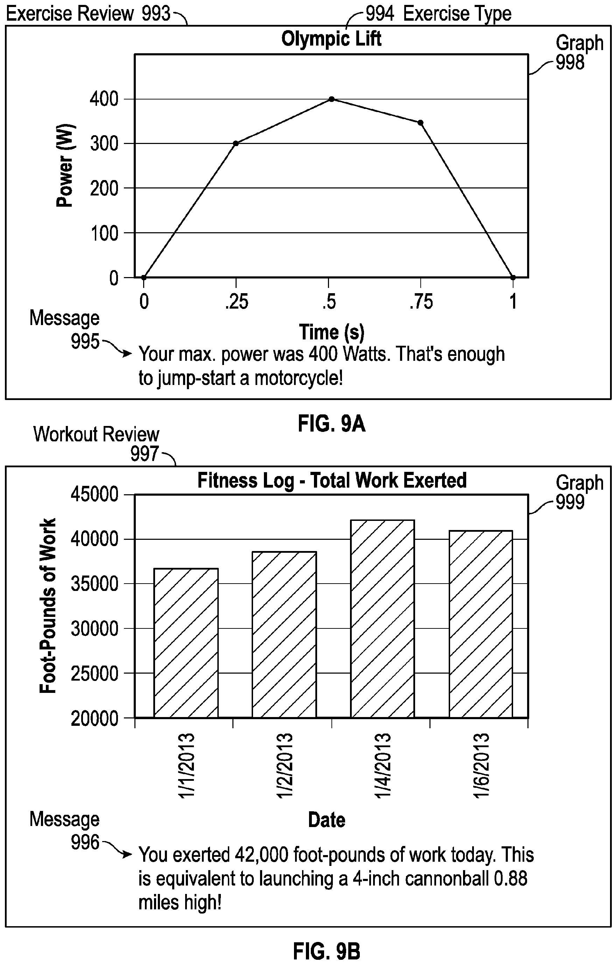

FIG. 9A illustrates exemplary exercise review 993 of a tracked exercise. Exercise review 993 can be displayed on a user device, on a computer monitor, on a web interface, on a display incorporated into a sensor device, or the like. As mentioned above, a sensor network can be used to recognize physical activities and track a user's workout, including strength training exercises. Exercise review 993 can display a visualization of a particular exercise, and specifically how a user performed during the exercise. For example, exercise review 993 can include an indication of a particular exercise type 994 along with graph 998 and message 995.

In one example, exercise type 994 can include an Olympic lift. Graph 998 can include a variety of information related to a user's performance of a particular exercise, such as the amount of power exerted over time during an exercise. For example, graph 998 in the illustrated example depicts the power a user exerted in watts during a one-second time period. Message 995 can include a variety of information, such as an exercise summary, statistics, a motivational phrase, or the like. For example, message 995 in the illustrated example notes that the user's maximum power during the Olympic lift was four hundred watts. In addition, message 995 can also include a motivational phrase, such as indicating that the amount of power exerted is sufficient to jump-start a motorcycle. Other motivational phrases can also be included that can compare exerted power to other applications. A variety of other messages and informational phrases can also be included in message 995. Graph 998 can also include a variety of other information as desired for different exercises.

FIG. 9B illustrates exemplary workout review 997 including a fitness log tracking total work exerted during different workouts. Workout review 997 can be displayed on a user device, on a computer monitor, on a web interface, on a display incorporated into a sensor device, or the like. As mentioned above, a sensor network can be used to recognize physical activities and track a user's workouts. Workout review 997 can display a visualization of workouts over time or a fitness log depicting workout performance on different occasions.

Workout review 997 can include a variety of information summarizing a user's performance during a number of prior workouts. Workout review 997 can include, for example, graph 999 to graphically depict performance as well as message 996 to summarize. In one example, graph 999 can include a bar graph depicting the foot-pounds of work exerted during workouts on different days. Other visualizations are also possible for graphically depicting workout performance on different occasions. Workout review 997 can also include message 996, which can include a variety of information, such as a workout summary, statistics, a motivational phrase, or the like. For example, message 996 can include a message indicating that a user exerted a certain amount of work during a particular workout. In addition, message 996 can include a motivational message comparing the exerted work to another application, such as how high a cannonball can be launched given the amount of work exerted. A variety of other messages and informational phrases can also be included in message 996. Graph 999 can also include a variety of other information as desired for depicting workout performance over time.

It should be understood that the exercise and workout reviews illustrated in FIG. 9A and FIG. 9B are examples of a variety of visualizations that can be provided to a user based on tracked exercises and workouts. It should likewise be understood that different types of reviews, graphs, and visualizations can be used for different exercise types, and that the metrics and units of measure for different exercises and workouts can be altered as desired.

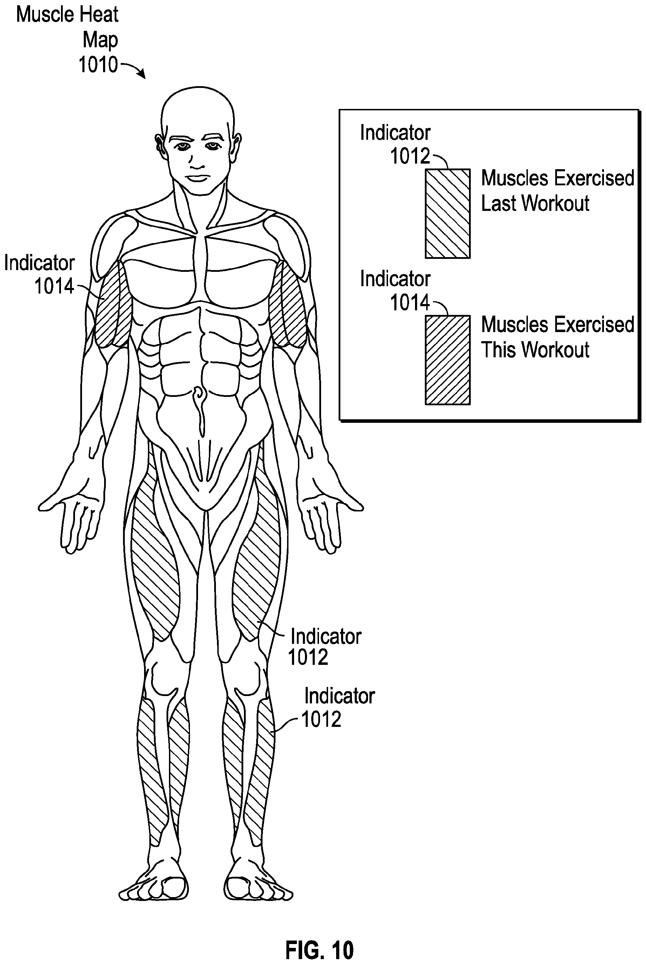

FIG. 10 illustrates exemplary muscle heat map 1010 indicating muscles exercised during different workouts. As with the exercise and workout reviews depicted in FIG. 9A and FIG. 9B, muscle heat map 1010 can be displayed on a user device, on a computer monitor, on a web interface, on a display incorporated into a sensor device, or the like. Likewise, muscle heat map 1010 can be generated based on physical activities and workouts recognized and tracked using a sensor network as discussed herein. Muscle heat map 1010 can include a map of muscles on a human figure along with a variety of information correlating particular muscles with exercises or workouts. In one example, muscle heat map 1010 can graphically illustrate muscles that a user exercised in a workout based on tracked activities and exercises. A database can be referenced that correlates particular exercises with particular muscles to determine which muscle areas should be highlighted. For example, indicator 1012 can be overlaid on particular muscles that were exercised in a previous workout, such as particular leg muscles that were exercised from one or more leg exercises performed in a previous workout.

In another example, muscles exercised during different workouts can be depicted on the same muscle heat map. For example, indicator 1014 can be overlaid on muscles exercised in a recent workout, such as particular arm muscles that were exercised from one or more arm exercises performed in a recent workout. In some examples, muscles emphasized or highlighted with an indicator can be selected by a user, and corresponding exercises, fitness logs, workout summaries, or the like can be displayed indicating why those muscles were highlighted. In other examples, any muscle can be selected by a user, and corresponding exercises or physical activities can be displayed indicating how those particular muscles can be exercised.

Although illustrated using a pattern, indicators 1012 and 1014 can include colors, shading, patterns, texture, animations, or the like for highlighting exercised muscles. In addition, indicators 1012 and 1014 can change over time based on muscle recovery rates, workout intensity, workout duration, or the like, and such a time-variant display can be based on information from a database of muscle recovery times compared to a user's particular workouts and/or a user's personal characteristics. For example, muscles that were strenuously exercised very recently can be highlighted in red to indicate, for example, that those muscles are likely still recovering from the strenuous exercise (e.g., those muscles are "hot"). In contrast, muscles that were moderately exercised or exercised many days earlier can be highlighted in green or blue to indicate, for example, that those muscles are likely mostly recovered from the moderate or more distant exercise (e.g., those muscles are "cool").

Muscle heat map 1010 can also be used to make suggestions to a user based on workout history and potential exercises. In one example, muscles that have not been exercised recently can be shaded gray, for example, to indicate they may be dormant or can be highlighted in yellow, for example, to indicate that it may be desirable to focus on those areas given the user's workout history. Selecting those suggested muscle areas can, in some examples, cause a list of suggested exercises to be provided to the user for exercising the highlighted muscle areas. In this manner, muscles throughout a user's body can be monitored based on tracked physical activities, and meaningful suggestions can be provided for optimizing subsequent workouts to, among other things, exercise ignored muscle areas, allow for desirable recovery times for recently exercised muscles, and the like. Moreover, the visualization provided by muscle heat map 1010 can provide users with motivation and help users set workout goals (e.g., keep all muscle areas in certain shades, avoid ignoring certain muscle areas, respect muscle recover times, etc.).

It should be understood that many variations are possible for muscle heat map 1010. For example, the human figure can be rotatable to allow users to monitor muscles all around the body. Similarly, the human figure can be tailored to a particular user's physical characteristics (e.g., similar gender, height, and proportions). In some examples, sensors as discussed herein can be used to detect muscle strain that can be depicted visually in muscle heat map 1010, or a user can manually input information about muscle status (e.g., muscle soreness, strain, etc.) that can be visually reproduced in muscle heat map 1010. Still other variations are possible in collecting information and visually depicting it in muscle heat map 1010.

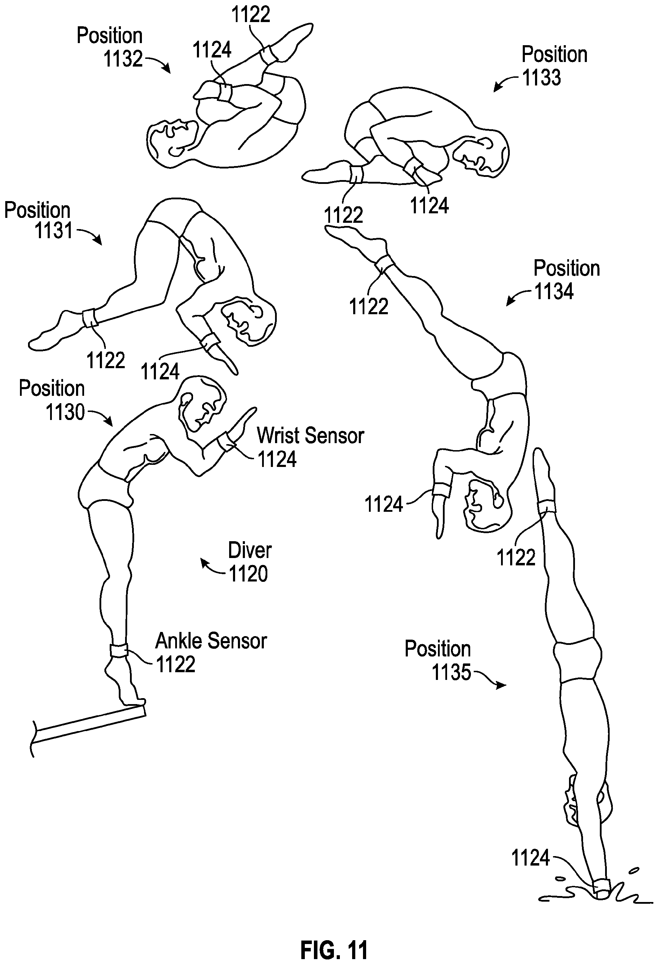

FIG. 11 illustrates an exemplary sensor network including wrist sensor 1124 and ankle sensor 1122 on diver 1120 to track the diver's position during a front flip dive. As mentioned above, the various sensors and devices discussed herein can be used to recognize, track, and even record in three dimensions a user's physical activities. Such physical activities can include dance routines, exercises, sporting activities, or the like, including diving. In some examples, the various sensors discussed herein can be waterproof or otherwise safely usable in a wet environment. FIG. 11 illustrates how a sensor network combination of wrist sensor 1124 and ankle sensor 1122 can be used to track the body position, orientation, and the like of diver 1120 for a variety of purposes, such as subsequent analysis, entertainment, replaying, receiving feedback on improving, or the like.

Although a single ankle sensor 1122 and single wrist sensor 1124 are shown, it should be understood that other sensors can also be included in the illustrated sensor network, such as an addition ankle sensor on the other ankle, an additional wrist sensor on the other wrist, head sensors, core sensors, arm sensors, or the like. In some examples, additional sensors can provide enhanced tracking accuracy. In addition, although a user device (e.g., a smartphone) is not shown, it should be understood that a user device (which can be waterproof in some examples) can also be worn by diver 1120 in an armband or the like (which can also provide waterproof protection for the device). In other examples, however, a user device in communication with ankle sensor 1122 and wrist sensor 1124 can be located nearby (e.g., on the pool deck), and the user device and sensors can include a communication means with sufficient range so as to allow the sensors to provide sensor data to the user device without diver 1120 carrying the user device during the dive (e.g., Bluteooth, Wi-Fi, RF, or other communication means with sufficient range).

In still other examples, ankle sensor 1122 and wrist sensor 1124 can include memories that can record sensor data during the dive. The recorded data in the memories can then be transmitted to a user device at a later time. For example, ankle sensor 1122 and wrist sensor 1124 can record sensed data throughout the dive, and the recorded data can be transferred to a user device after diver 1120 exits the pool and the sensors are positioned sufficiently near the user device for communication (e.g., within communication range). The user device can receive the recorded data and process it to provide the desired information to the user, such as a three-dimensional recording of the dive.

Ankle sensor 1122 and wrist sensor 1124 can include a variety of sensors as discussed above that can enable tracking of a variety of information, such as the distance between the sensors, the relative position of the sensors compared to a fixed reference (e.g., the ground, a magnetic pole, a starting position, etc.), the movement of the sensors in three dimensional space, the angular acceleration of the sensors, the angle of the wrist relative to a fixed reference, the angle of the ankle relative to a fixed reference, or the like. Other sensors can also be included for tracking other data, such as the diver's heart rate, the environmental temperature, the humidity, and the like.

During the dive, ankle sensor 1122 and wrist sensor 1124 can detect motion information and other data sufficient to map the path of the diver in three-dimensional space. Beginning at position 1130, ankle sensor 1122 and wrist sensor 1124 can detect (or sensed data can be used to infer) that the ankle is below the wrist, that they are spaced apart such that the diver's arm is raised above the chest (e.g., based on prior data collected while walking or performing a training or calibration sequence to determine expected hand positions, user height, etc.), and that the wrist is quickly moving downward in an arc. At position 1131, ankle sensor 1122 and wrist sensor 1124 can detect that the sensors are close together to infer that the body is bent as well as detect that both sensors are moving in a clockwise arc at a similar velocity. At position 1132, the sensors are brought even closer together, and the sensed data can enable a determination that diver 1120 is more tightly bent or crouched tightly. The detected clockwise arc motion is continued at position 1133, and ankle sensor 1122 and wrist sensor 1124 can detect that total forward movement has been greater than rearward movement, such that it can be determined that diver 1120 has traveled forward in space as illustrated.

At position 1134, ankle sensor 1122 and wrist sensor 1124 can detect that the distance between the devices is increasing, such that it can be determined that diver 1120 is releasing out of the crouched or bent position. In addition, it can be detected that both sensors are moving downward, and that the wrist sensor is below the ankle sensor, such that it can be determined that diver 1120 is in a head-first dive compared to the feet-first starting position. At position 1135, ankle sensor 1122 and wrist sensor 1124 can detect a maximum separation between the devices, such that it can be determined that diver 1120 has his hands outstretched well above his head and his legs pointed straight. For example, the diver's height and expected arm length can be determined from a calibration sequence prior to the dive, so the diver's stance at position 1135 can be determined based on the limits of arm and leg length. Subsequent to position 1135, in some examples, the sensors can detect entry into the water at different times, which can suggest a location of the water relative to the starting position as well as confirm the deceleration sensed as the diver enters the water and slows from free fall.

In some examples, the position of the diver's core or head can be indeterminate based on ankle and wrist sensor data alone. In such instances, analysis software can infer from the data the most likely stance or position of the diver based, for example, on models accounting for the typical limits of human movement (e.g., limits of bending). In other examples, software can offer a user various possibilities and selectable options for resolving any ambiguities while reviewing the recorded data. In addition, as mentioned above, additional sensors can be provided as desired to improve resolution and the ability of analysis software to determine a user's stance and movements (e.g., head sensors, core sensors, etc.).

The three-dimensional recording illustrated in FIG. 11 can be provided to a user in a variety of ways for analysis and activity tracking, such as in an animation (e.g., a virtual playback), a time-stop or stop-motion image similar to FIG. 11, or the like. Subsequent repetitions of the same or a similar activity can also be compared to monitor improvement. For example, data from subsequent dives can be compared to the data corresponding to FIG. 11 to compare the diver's form, timing, path traveled, and the like. Although the example of a front flip dive has been described, it should be understood that such activity monitoring and tracking can be performed for a variety of other physical activities with a variety of sensor combinations as desired.

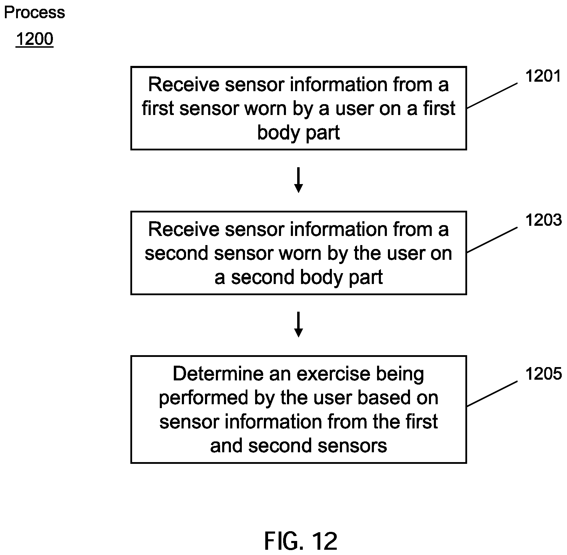

FIG. 12 illustrates exemplary process 1200 for determining an exercise being performed by a user from sensor information. At block 1201, sensor information can be received from a first sensor worn by a user on a first body part. The first sensor can include any of the sensors in any of the placements discussed herein. For example, the first sensor can include an ankle sensor, a wrist sensor, a headphone sensor, an armband sensor, a shoe sensor, a sensor in a smartphone, a sensor in a media player, or the like. A user can indicate the placement of the sensor via an app on a user device, via an interface element on the sensor device, or the like. In one example, the placement of the sensor can be automatically determined from recognized movements during, for example, a calibration or training period (e.g., recognizing typical wrist motions, arm motions, foot motions, head motions, or the like while a user is walking).

Sensor information received at block 1201 can include any of a variety of sensed information discussed herein. For example, sensor information can include motion information from accelerometers, gyroscopes, or the like. Sensor information can also include positional information, such as GPS data, magnetometer readings, or the like. Sensor information can also include various other sensor readings and data as discussed herein.

At block 1203, sensor information can be received from a second sensor worn by the user on a second body part. As with the first sensor, the second sensor can include any of the sensors in any of the placements discussed herein. For example, the second sensor can include an ankle sensor, a wrist sensor, a headphone sensor, an armband sensor, a shoe sensor, a sensor in a smartphone, a sensor in a media player, or the like. In some examples, the second sensor can be positioned on a different body part type than the first sensor. For example, if the first sensor is a shoe, foot, or ankle sensor, the second sensor can be positioned on a user's wrist, arm, hip, head, or the like. In other examples, however, sensors on both ankles, both shoes, both wrists, both arms, or the like can be used.

As with the fist sensor, sensor information received at block 1203 can include any of a variety of sensed information discussed herein. In some examples, the first and second sensors can work in a coordinated manner to provide sensor data relative to one another. For example, the first and second sensors can provide a distance between the two sensors, relative angles between the two sensors, relative orientations between the two sensors, or the like.

At block 1205, an exercise being performed by the user can be determined based on the sensor information received from the first and second sensors. For example, sensor information received from the first and second sensors can be used to determine that a user is performing jumping jacks, sit-ups, chin ups, pull ups, dips, lateral pull-downs, overhead shoulder presses, bent-over barbell rows, bent-over dumbbell rows, upright rows, cable rows, barbell bench presses, dumbbell bench presses, pushups, squats, lunges, deadlifts, power cleans, back extensions, or the like. In some examples, the received sensor information can be compared to a database of recognized exercise types to automatically determine which exercise the user is performing (in some examples, without any other input from the user). The user's prior exercise history and recognized movements can also be used to determine which exercise the user is likely performing (e.g., recognizing that previously recognized or identified exercises can be more likely). In some examples, users can perform new motions or exercises not yet in the database (e.g., not yet automatically recognizable) and provide for future recognition of the new motions or exercises. For example, a user can perform a motion and manually identify the associated exercise or a name for the performed motion (e.g., an unrecognized martial arts movement). A user device, server, or the like can store the sensor information received while the user performed the motion and compare future movements to the stored information to automatically recognize the identified exercise or named motion in the future.

In some examples, the recognized exercise can be recorded and tracked as part of a fitness log or workout history. A variety of information can be recorded and associated with a recognized exercise. For example, the number of repetitions, the duration, the acceleration, the date, the time of day, or the like can be recorded for a recognized exercise. Different exercises can also be recognized and recorded to track an entire workout, such as monitoring and recording all sets and all repetitions of different exercises during a workout. The recorded information can be used to display comparisons, progress, performance, and other information. In some examples, exercise summaries, workout summaries, muscle heat maps, and the like can be generated and displayed based on the recognized exercises and recorded exercise information.

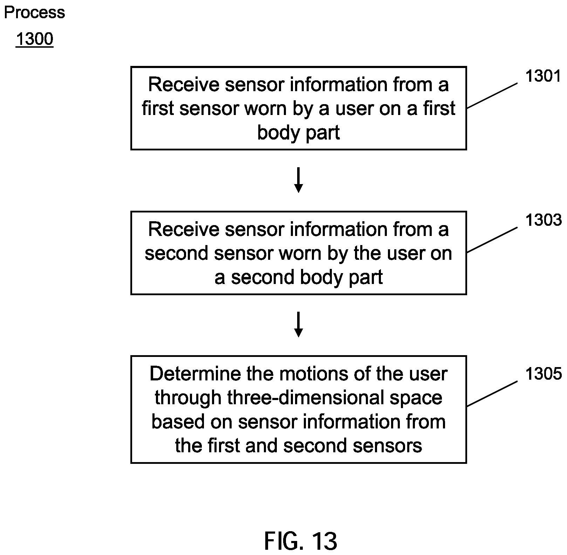

FIG. 13 illustrates exemplary process 1300 for determining the motions of a user through three-dimensional space from sensor information. Such three-dimensional motion recording can be used to track a variety of user motions for subsequent review, analysis, tracking, or the like. For example, a user's dance routine, martial arts routine, gymnastics routine, dive, ski jump, trampoline activity, golf swing, bat swing, basketball shot, running form, various other sports motions, various other performance motions, various other exercise activity motions, and the like can be monitored and recorded for subsequent analysis, for entertainment, for progress tracking, for record-keeping, for a fitness log, or the like.

At block 1301, sensor information can be received from a first sensor worn by a user on a first body part. The first sensor can include any of the sensors in any of the placements discussed herein, and sensor information received at block 1301 can include any of a variety of sensed information discussed herein.

At block 1303, sensor information can be received from a second sensor worn by the user on a second body part. As with the first sensor, the second sensor can include any of the sensors in any of the placements discussed herein, and sensor information received at block 1303 can include any of a variety of sensed information discussed herein. In some examples, the first and second sensors can work in a coordinated manner to provide sensor data relative to one another. For example, the first and second sensors can provide a distance between the two sensors, relative angles between the two sensors, relative orientations between the two sensors, or the like.

At block 1305, motions of the user through three-dimensional space can be determined based on the sensor information from the first and second sensors. For example, sensor information received from the first and second sensors can be used to determine that a user is spinning during a dance routine, performing a front flip during a dive, kicking at a certain height during a martial arts routine, traveling at a certain rate across a floor mat during a gymnastic routine, swinging an arm at an odd angle during a golf swing, or any of a variety of other motions through three-dimensional space. In some examples, sufficient data can be gathered from the sensors to map the movement of a user's body through three-dimensional space over time, such as, for example, mapping the movement of a user's body in three-dimensional space throughout a dive (e.g., using three-dimensional coordinates, tracking displacement through three-dimensional space, etc.).

In some examples, a user can wear additional sensors on other body parts, and the additional sensor information can allow for enhanced resolution, detail, or accuracy in the recognized motions through three-dimensional space. For example, while the position of a user's head can be inferred from the limits of human motion, in some examples a more detailed record of head movements can be desirable. In such an instance, one or more head sensors can be worn by the user (e.g., in headphones, a headband, earrings, or the like). The sensed information from the head sensor or head sensors can then be used to more accurately determine the motion of the user's head while also determining the motions of the rest of the user's body. Additional sensors can likewise be worn on other portions of the body for more accurate tracking as desired. For example, for detailed tracking of arm movements in a punching motion, multiple sensors can be worn on a user's arm (e.g., near the shoulder, at the elbow, at the wrist, on the hand, etc.). In other examples, multiple sensors can be placed in other positions on a user's body to improve accuracy as desired.

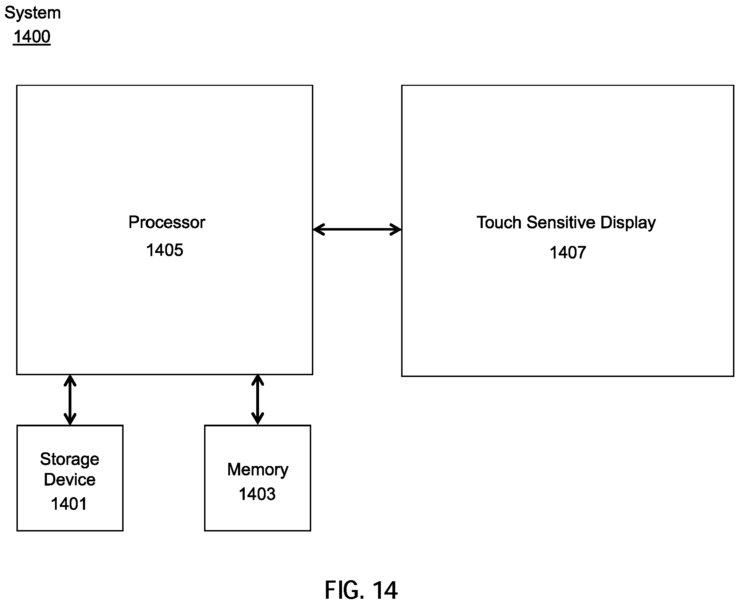

One or more of the functions described above relating to receiving and processing sensor information can be performed by a system similar or identical to system 1400 shown in FIG. 14. System 1400 can include instructions stored in a non-transitory computer readable storage medium, such as memory 1403 or storage device 1401, and executed by processor 1405. The instructions can also be stored and/or transported within any non-transitory computer readable storage medium for use by or in connection with an instruction execution system, apparatus, or device, such as a computer-based system, processor-containing system, or other system that can fetch the instructions from the instruction execution system, apparatus, or device and execute the instructions. In the context of this document, a "non-transitory computer readable storage medium" can be any medium that can contain or store the program for use by or in connection with the instruction execution system, apparatus, or device. The non-transitory computer readable storage medium can include, but is not limited to, an electronic, magnetic, optical, electromagnetic, infrared, or semiconductor system, apparatus or device, a portable computer diskette (magnetic), a random access memory (RAM) (magnetic), a read-only memory (ROM) (magnetic), an erasable programmable read-only memory (EPROM) (magnetic), a portable optical disc such as CD, CD-R, CD-RW, DVD, DVD-R, or DVD-RW, or flash memory such as compact flash cards, secured digital cards, USB memory devices, memory sticks, and the like.

The instructions can also be propagated within any transport medium for use by or in connection with an instruction execution system, apparatus, or device, such as a computer-based system, processor-containing system, or other system that can fetch the instructions from the instruction execution system, apparatus, or device and execute the instructions. In the context of this document, a "transport medium" can be any medium that can communicate, propagate, or transport the program for use by or in connection with the instruction execution system, apparatus, or device. The transport medium can include, but is not limited to, an electronic, magnetic, optical, electromagnetic, or infrared wired or wireless propagation medium.

System 1400 can further include touch sensitive display 1407 coupled to processor 1405 for detecting touch and displaying information. It is to be understood that the system is not limited to the components and configuration of FIG. 14, but can include other or additional components in multiple configurations according to various examples. Additionally, the components of system 1400 can be included within a single device, or can be distributed between multiple devices. In some examples, processor 1405 can be located within touch sensitive display 1407.

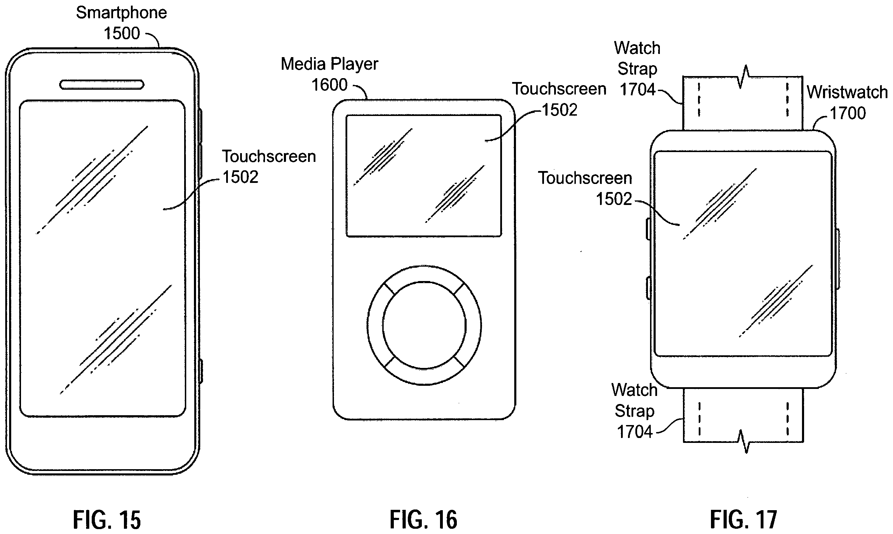

FIG. 15 illustrates exemplary smartphone 1500 that can receive and process sensor information according to various examples herein. In some examples, smartphone 1500 can include touchscreen 1502 for detecting touch and displaying information.

FIG. 16 illustrates exemplary media player 1600 that can receive and process sensor information according to various examples herein. In some examples, media player 1600 can include touchscreen 1502 for detecting touch and displaying information.

FIG. 17 illustrates exemplary wristwatch 1700 that can receive and process sensor information according to various examples herein. In some examples, wristwatch 1700 can include touchscreen 1502 for detecting touch and displaying information. Wristwatch 1700 can also include watch strap 1704 for securing wristwatch 1700 to a user's wrist. In some examples, wristwatch 1700 can include a variety of sensors as discussed herein and can function in a sensor network in conjunction with a user device, such as smartphone 1500 of FIG. 15.

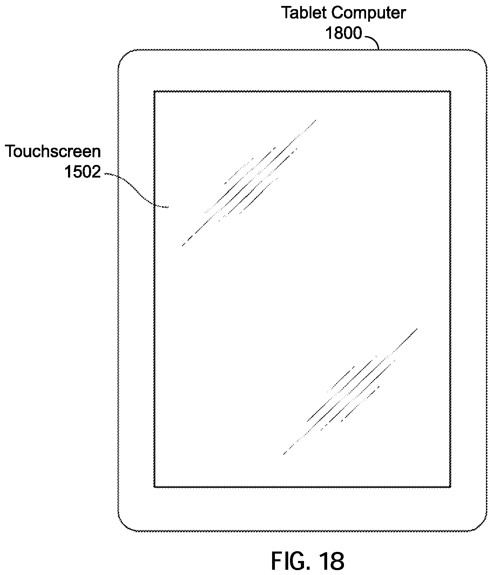

FIG. 18 illustrates exemplary tablet computer 1800 that can receive and process sensor information according to various examples herein. In some examples, tablet computer 1800 can include touchscreen 1502 for detecting touch and displaying information.

Therefore, according to the above, some examples of the disclosure are directed to a sensor network comprising: a first sensor capable of being secured proximate to a first part of a body of a user; a second sensor capable of being secured proximate to a second part of the body of the user; and a user device capable of receiving sensor information from the first and second sensors and determining a physical activity of the user based on the sensor information. Additionally or alternatively to one or more of the examples disclosed above, in some examples the physical activity of the user comprises an exercise performed by the user. Additionally or alternatively to one or more of the examples disclosed above, in some examples the physical activity of the user comprises a stance of the user. Additionally or alternatively to one or more of the examples disclosed above, in some examples the physical activity of the user comprises motions of the user through three-dimensional space. Additionally or alternatively to one or more of the examples disclosed above, in some examples the first sensor comprises a wrist sensor; and the wrist sensor is capable of generating sensor information comprising data indicating movement of a wrist of the user. Additionally or alternatively to one or more of the examples disclosed above, in some examples the second sensor comprises an ankle sensor or a shoe sensor; and the ankle sensor or the shoe sensor is capable of generating sensor information comprising data indicating movement of an ankle or a foot of the user.

According to the above, other examples of the disclosure are directed to a method for sensing a physical activity of a user, comprising: receiving a first signal from a first sensor proximate to a first body part of a user, wherein the first signal includes first information about the first body part; receiving a second signal from a second sensor proximate to a second body part of the user, wherein the second signal includes second information about the second body part; and determining a physical activity of the user based on the received first and second signals. Additionally or alternatively to one or more of the examples disclosed above, in some examples determining the physical activity of the user comprises: determining an exercise of the user; wherein the first information comprises at least one of a position or a motion of the first body part; and wherein the second information comprises at least one of a position or a motion of the second body part. Additionally or alternatively to one or more of the examples disclosed above, in some examples determining the physical activity of the user comprises: determining a motion of the user through three-dimensional space; wherein the first information comprises a displacement through three-dimensional space of the first body part; and wherein the second information comprises a displacement through three-dimensional space of the second body part. Additionally or alternatively to one or more of the examples disclosed above, in some examples determining the physical activity of the user comprises: determining a stance of the user; wherein the first information comprises a position of the first body part; and wherein the second information comprises a position of the second body part. Additionally or alternatively to one or more of the examples disclosed above, in some examples determining the physical activity of the user comprises: comparing the first information and the second information to a database to determine an exercise being performed by the user, wherein the database comprises one or more exercises correlated with expected sensor information. Additionally or alternatively to one or more of the examples disclosed above, in some examples the method for sensing a physical activity of a user further comprises: recording a number of repetitions of the determined exercise performed by the user. Additionally or alternatively to one or more of the examples disclosed above, in some examples the method for sensing a physical activity of a user further comprises: causing a fitness log to be displayed, wherein the fitness log comprises a graph reflecting the recorded number of repetitions of the determined exercise performed by the user.

According to the above, other examples of the disclosure are directed to a user device comprising: a receiver capable of receiving a first signal from a first sensor worn on a first body part of a user and a second signal from a second sensor worn on a second body part of the user, the first and second signals indicating sensor information about the first and second body parts; and a processor capable of analyzing the first and second signals to determine a physical activity of the user. Additionally or alternatively to one or more of the examples disclosed above, in some examples the sensor information indicates a movement of the first body part through three-dimensional space and a movement of the second body part through three-dimensional space; and wherein the user device is capable of recording the movement of the first body part through three-dimensional space and the movement of the second body part through three-dimensional space. Additionally or alternatively to one or more of the examples disclosed above, in some examples the user device is further capable of causing to be displayed a virtual playback of the recorded movement of the first body part through three-dimensional space and the recorded movement of the second body part through three-dimensional space. Additionally or alternatively to one or more of the examples disclosed above, in some examples the receiver is further capable of receiving a third signal from a third sensor worn on a third body part of the user, the third signal indicating sensor information about the third body part; and the sensor information about the third body part indicates a movement of the third body part through three-dimensional space.

According to the above, other examples of the disclosure are directed to a sensor network comprising: multiple sensors capable of being secured proximate to different body parts of a user, the sensors capable of sensing information about the different body parts; and a processor capable of receiving the sensed information about the different body parts from the multiple sensors and determining a physical activity of the user based on the sensed information. Additionally or alternatively to one or more of the examples disclosed above, in some examples the sensed information indicates movements of the different body parts through three-dimensional space; and the processor is capable of causing to be recorded the movements of the different body parts through three-dimensional space. Additionally or alternatively to one or more of the examples disclosed above, in some examples the processor is further capable of causing to be displayed a virtual playback of the recorded movements of the different body parts through three-dimensional space.

According to the above, other examples of the disclosure are directed to a method comprising: receiving sensor information from a first sensor device worn by a user on a first body part; receiving sensor information from a second sensor device worn by the user on a second body part; determining an exercise being performed by the user based on the sensor information from the first and second sensors; and storing a number of repetitions of the determined exercise performed by the user. Additionally or alternatively to one or more of the examples disclosed above, in some examples the method further comprises: determining muscles exercised based on the determined exercise performed by the user; and causing to be displayed a muscle heat map, wherein the muscle heat map comprises a display of multiple muscles of a body, and wherein the muscle heat map graphically indicates which muscles of the multiple muscles were determined to have been exercised. Additionally or alternatively to one or more of the examples disclosed above, in some examples the method further comprises: causing a display of the first sensor device to be enabled based on the received sensor information from the first sensor device comprising data indicating a movement of the first sensor device toward a face of the user. Additionally or alternatively to one or more of the examples disclosed above, in some examples the method further comprises: causing a vibrator of the first sensor device to vibrate based on the received sensor information from the first sensor device comprising data indicating one or more of completion of a set of exercise repetitions, an exercise pace being outside a designated range, reaching an extreme limit of an exercise motion, or a heart rate of the user being outside a designated range. Additionally or alternatively to one or more of the examples disclosed above, in some examples the method further comprises: receiving data from a communication tag associated with a piece of exercise equipment; and storing the received data with the stored number of repetitions of the determined exercise performed by the user.

According to the above, other examples of the disclosure are directed to a sensor network comprising: a sensor capable of being secured proximate to a part of a body of a user; and a user device capable of receiving sensor information from the sensor and determining a physical activity of the user based on the sensor information.

Although examples have been fully described with reference to the accompanying drawings, it is to be noted that various changes and modifications will become apparent to those skilled in the art. Such changes and modifications are to be understood as being included within the scope of the various examples as defined by the appended claims.

## Claims

1. A method for sensing a physical activity of a user, comprising:
receiving a first signal from a first wearable sensor configured to be worn proximate to a first body part of the user, wherein the first signal includes first information about the first body part comprises at least one of a position or a motion of the first body part;
receiving a second signal from a second wearable sensor configured to be worn proximate to a second body part of the user, wherein the second signal includes second information about the second body part comprises at least one of a position or a motion of the second body part; **characterised by**:
receiving a third signal from a sensor or a tag of non-wearable equipment comprising an exercise machine, wherein the third signal includes third information about the equipment comprising a type of exercise machine; and
determining a physical activity of the user based on the received first, second and third signals, wherein determining the physical activity of the user comprises determining an exercise performed by the user.

2. The method of claim 1, wherein determining the physical activity of the user comprises:
determining a motion of the user through three-dimensional space;
wherein the first information comprises a displacement through three-dimensional space of the first body part; and
wherein the second information comprises a displacement through three-dimensional space of the second body part.

3. The method of claim 1, wherein determining the physical activity of the user comprises:
determining a stance of the user;
wherein the first information comprises the position of the first body part; and
wherein the second information comprises the position of the second body part.

4. The method of claim 1, wherein determining the physical activity of the user comprises:
comparing the first information and the second information to a database to determine an exercise being performed by the user, wherein the database comprises one or more exercises correlated with expected sensor information.

5. The method of claim 1, further comprising:
recording a number of repetitions of the determined exercise performed by the user.

6. The method of claim 5, further comprising:
causing a fitness log to be displayed, wherein the fitness log comprises a graph reflecting the recorded number of repetitions of the determined exercise performed by the user.

7. The method of claim 1, further comprising:
scanning the sensor or the tag of the equipment with the first wearable sensor, the second wearable sensor or a user device; and
receiving the third information from the sensor or the tag of the equipment in response to the scanning.

8. The method of claim 1, wherein the third information from the sensor or the tag of the equipment corresponds to the sensor or tag of the equipment nearest to the first wearable sensor, the second wearable sensor or the user device receiving the third information.

9. The method of claim 1, further comprising:
in response to receiving the third information, causing playback of instructions or tips corresponding to the type of exercise machine.

10. The method of claim 1, further comprising:
in response to receiving the third information, causing display or playback of information regarding previous usage of the exercise machine by the user.

11. The method of claim 1, further comprising:
in response to receiving the third information, causing transmission of user setting to the exercise machine to adjust one or more of a selected weight, seat position or handle position corresponding to the exercise machine.

12. The method of claim 1, wherein the first sensor comprises a wrist sensor and the second sensor comprises an ankle sensor or a shoe sensor;
wherein the wrist sensor is capable of generating sensor information comprising data indicating movement of a wrist of the user; and
wherein the ankle sensor or the shoe sensor is capable of generating sensor information comprising data indicating movement of an ankle or a foot of the user.

13. A non-transitory computer readable storage medium, the computer readable storage medium containing instructions that, when executed, perform a method according to any of claims 1 to 12.

14. An electronic device configured to perform a method according to any of claims 1 to 12.

## Patentansprüche

1. Verfahren zum Erfassen einer körperlichen Aktivität eines Benutzers, umfassend:
Empfangen eines ersten Signals von einem ersten tragbaren Sensor, der so konfiguriert ist, dass er in der Nähe eines ersten Körperteils des Benutzers getragen wird, wobei das erste Signal erste Informationen über den ersten Körperteil enthält, die mindestens eine Position oder eine Bewegung des ersten Körperteils umfassen;
Empfangen eines zweiten Signals von einem zweiten tragbaren Sensor, der so konfiguriert ist, dass er in der Nähe eines zweiten Körperteils des Benutzers getragen wird, wobei das zweite Signal zweite Informationen über den zweiten Körperteil enthält, die mindestens eines von einer Position oder einer Bewegung des zweiten Körperteils umfassen; **gekennzeichnet durch**:
Empfangen eines dritten Signals von einem Sensor oder einem Etikett einer nicht tragbaren Ausrüstung, die ein Trainingsgerät umfasst, wobei das dritte Signal eine dritte Information über die Ausrüstung umfasst, die einen Typ eines Trainingsgeräts umfasst; und
Bestimmen einer körperlichen Aktivität des Benutzers auf der Grundlage des empfangenen ersten, zweiten und dritten Signals, wobei Bestimmen der körperlichen Aktivität des Benutzers Bestimmen einer von dem Benutzer durchgeführten Übung umfasst.

2. Verfahren nach Anspruch 1, wobei Bestimmen der körperlichen Aktivität des Benutzers umfasst:
Bestimmen einer Bewegung des Benutzers durch den dreidimensionalen Raum;
wobei die erste Information eine Verschiebung des ersten Körperteils durch den dreidimensionalen Raum umfasst; und
wobei die zweite Information eine Verschiebung des zweiten Körperteils durch den dreidimensionalen Raum umfasst.

3. Verfahren nach Anspruch 1, wobei Bestimmen der körperlichen Aktivität des Benutzers umfasst:
Bestimmen einer Haltung des Benutzers;
wobei die erste Information die Position des ersten Körperteils umfasst; und
wobei die zweite Information die Position des zweiten Körperteils umfasst.

4. Verfahren nach Anspruch 1, wobei Bestimmen der körperlichen Aktivität des Benutzers umfasst:
Vergleichen der ersten Information und der zweiten Information mit einer Datenbank, um eine Übung zu bestimmen, die von dem Benutzer durchgeführt wird, wobei die Datenbank eine oder mehrere Übungen umfasst, die mit erwarteten Sensorinformationen korreliert sind.

5. Verfahren nach Anspruch 1, ferner umfassend:
Aufzeichnen einer Anzahl von Wiederholungen der bestimmten Übung, die von dem Benutzer durchgeführt wird.

6. Verfahren nach Anspruch 5, ferner umfassend:
Veranlassen, dass ein Fitnessprotokoll angezeigt wird, wobei das Fitnessprotokoll einen Graphen umfasst, der die aufgezeichnete Anzahl von Wiederholungen der bestimmten, vom Benutzer durchgeführten Übung wiedergibt.

7. Verfahren nach Anspruch 1, ferner umfassend:
Scannen des Sensors oder des Etiketts der Ausrüstung mit dem ersten tragbaren Sensor, dem zweiten tragbaren Sensor oder einem Benutzergerät; und
Empfangen der dritten Information von dem Sensor oder dem Etikett der Ausrüstung als Reaktion auf das Scannen.

8. Verfahren nach Anspruch 1, wobei die dritte Information von dem Sensor oder dem Etikett der Ausrüstung dem Sensor oder dem Etikett des Ausrüstung entspricht, das dem ersten tragbaren Sensor, dem zweiten tragbaren Sensor oder dem Benutzergerät, das die dritte Information empfängt, am nächsten ist.

9. Verfahren nach Anspruch 1, ferner umfassend:
als Reaktion auf Empfangen der dritten Information, Veranlassen der Wiedergabe von Anweisungen oder Tipps, die dem Typ des Trainingsgeräts entsprechen.

10. Verfahren nach Anspruch 1, ferner umfassend:
als Reaktion auf Empfangen der dritten Information, Veranlassen der Anzeige oder Wiedergabe von Informationen bezüglich der vorherigen Benutzung des Trainingsgeräts durch den Benutzer.

11. Verfahren nach Anspruch 1, ferner umfassend:
als Reaktion auf Empfangen der dritten Information, Veranlassen der Übertragung einer Benutzereinstellung an das Trainingsgerät, um eines oder mehrere von einem ausgewählten Gewicht, einer Sitzposition oder einer Griffposition entsprechend dem Trainingsgerät einzustellen.

12. Verfahren nach Anspruch 1, wobei der erste Sensor einen Handgelenksensor umfasst und der zweite Sensor einen Knöchelsensor oder einen Schuhsensor umfasst; wobei der Handgelenksensor in der Lage ist, Sensorinformationen zu erzeugen, die Daten umfassen, die die Bewegung eines Handgelenks des Benutzers anzeigen; und wobei der Knöchelsensor oder der Schuhsensor in der Lage ist, Sensorinformationen zu erzeugen, die Daten umfassen, die die Bewegung eines Knöchels oder eines Fußes des Benutzers anzeigen.

13. Nicht-flüchtiges, computerlesbares Speichermedium, wobei das computerlesbare Speichermedium Anweisungen enthält, die, wenn sie ausgeführt werden, ein Verfahren nach einem der Ansprüche 1 bis 12 durchführen.

14. Elektronische Vorrichtung, die zur Durchführung eines Verfahrens nach einem der Ansprüche 1 bis 12 konfiguriert ist.

## Revendications

1. Un procédé de détection d'une activité physique d'un utilisateur, comprenant :
la réception d'un premier signal en provenance d'un premier capteur à porter sur soi configuré pour être porté à proximité d'une première partie du corps de l'utilisateur, le premier signal comprenant une première information relative à la première partie du corps, qui comprend au moins l'une d'entre une position ou un déplacement de la première partie du corps ;
la réception d'un second signal en provenance d'un second capteur à porter sur soi configuré pour être porté à proximité d'une seconde partie du corps de l'utilisateur, le second signal comprenant une seconde information relative à la seconde partie du corps, qui comprend au moins l'une d'entre une position ou un déplacement de la seconde partie du corps ;
**caractérisé par** :
la réception d'un troisième signal en provenance d'un capteur ou d'un badge d'un équipement ne pouvant pas être porté sur soi qui comprend une machine d'exercice, le troisième signal comprenant une troisième information relative à l'équipement, qui comprend un type de machine d'exercice ; et
la détermination d'une activité physique de l'utilisateur sur la base du premier, du second et du troisième signal reçus, la détermination de l'activité physique de l'utilisateur comprenant la détermination d'un exercice effectué par l'utilisateur.

2. Le procédé de la revendication 1, dans lequel la détermination de l'activité physique de l'utilisateur comprend :
la détermination d'un mouvement de l'utilisateur dans un espace tridimensionnel ;
dans lequel la première information comprend un déplacement de la première partie du corps dans l'espace tridimensionnel ; et
dans lequel la seconde information comprend un déplacement de la seconde partie du corps dans l'espace tridimensionnel.

3. Le procédé de la revendication 1, dans lequel la détermination de l'activité physique de l'utilisateur comprend :
la détermination d'une posture de l'utilisateur ;
dans lequel la première information comprend la position de la première partie du corps ; et
dans lequel la seconde information comprend la position de la seconde partie du corps.

4. Le procédé de la revendication 1, dans lequel la détermination de l'activité physique de l'utilisateur comprend :
la comparaison de la première information et de la seconde information à une base de données pour déterminer un exercice qui est effectué par l'utilisateur, la base de données comprenant un ou plusieurs exercices corrélés à une information de capteur prévue.

5. Le procédé de la revendication 1, comprenant en outre :
l'enregistrement d'un nombre de répétitions de l'exercice déterminé effectué par l'utilisateur.

6. Le procédé de la revendication 5, comprenant en outre :
l'affichage d'un journal de remise en forme, le journal de remise en forme comprenant un graphique reflétant le nombre de répétitions enregistré de l'exercice déterminé effectué par l'utilisateur.

7. Le procédé de la revendication 1, comprenant en outre :
la scrutation du capteur ou du badge de l'équipement avec le premier capteur à porter sur soi, le second capteur à porter sur soi ou un dispositif utilisateur ; et
la réception de la troisième information en provenance du capteur ou du badge de l'équipement en réponse à la scrutation.

8. Le procédé de la revendication 1, dans lequel la troisième information en provenance du capteur ou du badge de l'équipement correspond au capteur ou au badge de l'équipement le plus proche du premier capteur à porter sur soi, du second capteur à porter sur soi ou du dispositif utilisateur recevant la troisième information.

9. Le procédé de la revendication 1, comprenant en outre :
en réponse à la réception de la troisième information, la présentation d'instructions ou de conseils correspondant au type de machine d'exercice.

10. Le procédé de la revendication 1, comprenant en outre :
en réponse à la réception de la troisième information, l'affichage ou la restitution d'informations relatives à un usage antérieur de la machine d'exercice par l'utilisateur.

11. Le procédé de la revendication 1, comprenant en outre :
en réponse à la réception de la troisième information, la transmission à la machine d'exercice d'un paramétrage utilisateur pour ajuster un ou plusieurs d'entre un poids, une position de siège ou une position de poignet sélectionnés correspondant à la machine d'exercice.

12. Le procédé de la revendication 1, dans lequel le premier capteur comprend un capteur de poignet et le second capteur comprend un capteur de cheville ou un capteur de chaussure ;
dans lequel le capteur de poignet est capable de générer une information de capteur comprenant des données indiquant le mouvement d'un poignet de l'utilisateur ; et dans lequel le capteur de cheville ou le capteur de chaussure est capable de générer une information de capteur comprenant des données indiquant un mouvement d'une cheville ou d'un pied de l'utilisateur.

13. Un support de stockage non transitoire lisible par calculateur, le support de stockage lisible par calculateur contenant des instructions qui, lorsqu'elles sont exécutées, mettent en œuvre un procédé selon l'une des revendications 1 à 12.

14. Un dispositif électronique configuré pour mettre en œuvre un procédé selon l'une des revendications 1 à 12.
